# EUROPEAN PATENT APPLICATION

(11) **EP 4 850 617 A1**
(43) Date of publication of application: **09.12.2026**
(21) Application number: 25747902.2
(22) Date of filing: 24.01.2025
(51) Int. Cl.: A61B 17/16, A61B 17/14, A61B 17/32

(54) **RECIPROCATING CUTTING TOOL**

(30) Priority: 01.02.2024 CN 202420250138 U; 19.06.2024 CN 202421400600 U; 19.06.2024 CN 202421400605 U
(71) Applicant: Guizhou Zirui Technology Co., Ltd, Zunyi, Guizhou 563200 (CN)
(72) Inventor: LIU, Changfeng, Zunyi, Guizhou 563200 (CN); LI, Hongyuan, Zunyi, Guizhou 563200 (CN); LOU, Haiping, Zunyi, Guizhou 563200 (CN); LI, Huihua, Zunyi, Guizhou 563200 (CN); LINGHU, Feifei, Zunyi, Guizhou 563200 (CN); CHEN, Sihan, Zunyi, Guizhou 563200 (CN)
(74) Representative: Metida
(86) International application number: PCT/CN2025/075021
(87) International publication number: WO 2025/162334

(57) **Abstract**

The present invention provides a reciprocating surgical cutting instrument, comprising a cutter head assembly and a drive assembly. The drive assembly is drivingly connected to the cutter head assembly to drive the cutter head assembly to perform a reciprocating motion. By integrating the cutter head assembly and the drive assembly into a single product and powering it via an external drive member, the overall structure is compact and easy to maintain. Furthermore, it can convert the unidirectional motor rotation into reciprocating rotation, reciprocating oscillation, or linear reciprocating motion of the working portion of the cutter head assembly. Additionally, a water injection channel can be integrated into the product to flush and cool the cutter head assembly, thereby extending the service life of the cutter head assembly.

## Description

### TECHNICAL FIELD

The present invention relates to the technical field of medical instruments, and more particularly, to a reciprocating cutting instrument.

### BACKGROUND

At present, cutting instruments are commonly used in surgical procedures to perform grinding, shaving, resecting, or drilling operations on a patient's soft tissue, bone, or the like.

Chinese Patent Publication No. CN214017702U discloses a water-cooled reciprocating bur for surgical use. In this patent, a reciprocating assembly drives the bur to rotate reciprocally, and a cooling assembly reduces the temperature of the bur during bone tissue grinding. However, this design suffers from a complex drive structure; the motor and the transmission assembly are both housed within the housing, resulting in inconvenient maintenance.

Chinese Patent Publication No. CN205913375U discloses a surgical drill comprising a drill assembly and a reciprocating assembly. The reciprocating assembly, driven by a drive assembly, drives the drill assembly to perform a reciprocating rotational motion. The reciprocating assembly coupled to the shank drives the rotating bur to reciprocally grind a bone tissue under various operating conditions. A drawback of this configuration is the absence of a bur cooling system, leaving room for further improvement.

Chinese Patent Publication No. CN105832378B discloses a bone surgery drill comprising a bur and a shank connected to the bur. The bur is mounted through a support rod via the shank, and a support ring is provided on the support rod. This arrangement results in a transmission structure that is cumbersome to operate.

Chinese Patent Publication No. CN208769904U discloses an eccentric reciprocating surgical drill comprising a drill assembly and a reciprocating assembly. The drill assembly includes a bur and a support rod, wherein the bur is mounted through the support rod via the shank. A reciprocating assembly coupled to the shank drives the rotating bur to reciprocally grind a bone tissue. This reciprocating drill lacks a water-cooling system, leading to a short service life of the bur.

Chinese Patent Publication No. CN208769903U discloses a surgical drill with a reciprocating drive structure, comprising a drill assembly and a reciprocating assembly. The drill assembly includes a bur and a support rod, wherein the bur is mounted through the support rod via the shank. The reciprocating assembly coupled to the shank drives the rotating bur to reciprocally grind a bone tissue. This reciprocating drill lacks a water-cooling system, leading to a short service life of the bur.

Chinese Patent Publication No. CN209450602U discloses a drill for use in transforaminal endoscopic spinal surgery. The grinding depth of the bur is adjusted by manipulating the position of an operating sleeve. A reciprocating assembly coupled to the shank drives the rotating bur to reciprocally grind a bone tissue. The technical solution of this drill lacks a water-cooling system, leading to a short service life of the bur.

Currently, there exists an urgent market demand for an integrated product that stably converts the unidirectional rotation of a motor into a reciprocating motion of the cutter head.

### SUMMARY

In view of the drawbacks in the prior art, an object of the present invention is to provide a reciprocating cutting instrument.

According to one aspect of the present invention, a reciprocating cutting instrument comprises a cutter head assembly and a drive assembly, wherein the drive assembly is drivingly connected to the cutter head assembly and drives the cutter head assembly to perform a reciprocating motion.

According to another aspect of the present invention, a reciprocating cutting instrument comprises a housing, a cutter head assembly, and a drive assembly. The drive assembly comprises an eccentric shaft, an articulating connector, and a transmission rod. The eccentric shaft, the articulating connector, the transmission rod, and the cutter head assembly are all disposed within the housing, and a working end of the cutter head assembly extends out of the housing.

The articulating connector comprises a connecting frame and a swing rod, and is rotatably mounted in the housing. One end of the eccentric shaft extends into the connecting frame and is rotatably engaged therewith. The swing rod is hinged to one end of the transmission rod, and the other end of the transmission rod is drivingly connected to the cutter head assembly.

Rotation of the eccentric shaft drives the articulating connector to reciprocally swing about its hinge axis, and the swing rod of the articulating connector drives the transmission rod to perform a linear reciprocating motion along an axis of the transmission rod.

Preferably, the cutter head assembly includes a saw blade, a cutter head seat, and a connecting rod. One end of the connecting rod is coaxially fixed to the transmission rod, and the other end is coaxially fixed to the cutter head seat. The saw blade is fixedly mounted on an end of the cutter head seat distal to the connecting rod and extends out of the housing.

Preferably, a mounting sleeve is disposed at an end of the housing distal to the cutter head assembly, and the eccentric shaft and the articulating connector are both disposed in the mounting sleeve. A first water injection channel is formed between the mounting sleeve and the housing, a second water injection channel is formed between the connecting rod and the housing, and a third water injection channel is formed between the cutter head seat and the housing. The first, second, and third water injection channels are in sequential communication.

Preferably, a support sleeve is disposed between the housing and the connecting rod, and a sealing sleeve is fitted over an end of the support sleeve distal to the articulating connector to seal a junction between the support sleeve and the connecting rod.

Preferably, the saw blade has a uniform-thickness body. An axial mounting groove is formed on the cutter head seat, and one end of the saw blade is embedded and mounted in the axial mounting groove.

A rectangular facet is formed on an outer surface of the cutter head seat, and a connecting sleeve is disposed between the cutter head seat and a case. A third water injection channel is formed between the rectangular facet of the cutter head seat and the connecting sleeve.

According to another aspect of the present invention, a reciprocating cutting instrument comprises a housing, a cutter head assembly, and a drive assembly. The cutter head assembly includes an oscillating blade and an oscillating blade seat. The oscillating blade is fixedly connected to the oscillating blade seat, and the oscillating blade and/or the oscillating blade seat are hinged to the housing. An end of the oscillating blade distal to the oscillating blade seat extends out of the housing.

The drive assembly includes an eccentric shaft. A connecting frame is formed at an end of the oscillating blade seat distal to the oscillating blade. The eccentric shaft extends into the connecting frame and is rotatably engaged therewith. Rotation of the eccentric shaft drives the end of the oscillating blade extending out of the housing to reciprocally swing about a hinge axis.

Preferably, the drive assembly further includes a rotating shaft and a transmission shaft. The rotating shaft is coaxially fixed to the transmission shaft, and an end of the transmission shaft distal to the rotating shaft is coaxially fixed to an end of the eccentric shaft distal to the oscillating blade seat.

Preferably, a mounting sleeve is disposed at an end of the housing distal to the cutter head assembly, and the rotating shaft is disposed in the mounting sleeve. A structural support sleeve is disposed between the transmission shaft and the housing, and a sealing sleeve is disposed between the transmission shaft and the structural support sleeve.

A connecting sleeve is connected to an end of the structural support sleeve distal to the rotating shaft, and the eccentric shaft is rotatably disposed in the connecting sleeve.

Water injection channels in communication are formed between the mounting sleeve, the structural support sleeve, the connecting sleeve, and the housing, respectively. Openings of the water injection channels are located at an end of the connecting sleeve proximal to the cutter head assembly.

According to another aspect of the present invention, a reciprocating cutting instrument comprises a housing, a cutter head assembly, and a drive assembly. The cutter head assembly includes a trephine. The drive assembly includes an eccentric shaft, a transmission swing rod, and a central transmission shaft. The transmission swing rod includes a connecting frame and a transmission rod, with the connecting frame located at one axial end of the transmission rod. The eccentric shaft extends into the connecting frame and is rotatably engaged therewith. An end of the transmission rod distal to the connecting frame is coaxially fixed to one end of the central transmission shaft.

The other end of the central transmission shaft is fixedly connected to the trephine, and a working portion of the trephine extends out of the housing.

Rotation of the eccentric shaft drives the transmission swing rod to reciprocally swing, and the transmission swing rod drives the trephine to reciprocally rotate via the central transmission shaft.

Preferably, a mounting sleeve is disposed at an end of the housing distal to the cutter head assembly, and the eccentric shaft is disposed in the mounting sleeve. A structural support sleeve is disposed between the central transmission shaft and the housing, and a sealing sleeve is disposed between the central transmission shaft and the structural support sleeve.

A fail-safe sleeve is disposed between the central transmission shaft and the housing.

A water injection channel is formed between the mounting sleeve and the housing. A through hole communicating an inner cavity of the central transmission shaft with the water injection channel is formed on the central transmission shaft. A water injection hole is formed inside the trephine and communicates with the inner cavity of the central transmission shaft.

Preferably, a cutter head of the trephine is a hollow ring, and a weight-reduction window is formed in a body portion of the trephine.

According to another aspect of the present invention, a reciprocating cutting instrument comprises a housing, a cutter head assembly, and a drive assembly. The drive assembly includes an eccentric shaft, a transmission collar, an eccentric transmission shaft, and a connecting shaft. The cutter head assembly includes a grinding/milling cutter head. The transmission collar includes a first ring portion and a second ring portion. The first ring portion is fitted over the eccentric shaft and rotatably engaged therewith. The eccentric transmission shaft extends into the second ring portion and is rotatably engaged therewith. An end of the eccentric transmission shaft distal to the transmission collar is drivingly connected to the connecting shaft.

An end of the connecting shaft distal to the eccentric transmission shaft is drivingly connected to the grinding/milling cutter head.

Rotation of the eccentric shaft drives the eccentric transmission shaft to rotate via the transmission collar, thereby driving the grinding/milling cutter head to reciprocally rotate via the connecting shaft.

Preferably, grinding teeth are formed on both an end face and a side face of the grinding/milling cutter head, and a connecting arcuate surface is formed between the grinding teeth on the end face and the grinding teeth on the side face of the grinding/milling cutter head.

A central portion of the end face of the grinding/milling cutter head is concave.

Preferably, a mounting sleeve is disposed at an end of the housing distal to the cutter head assembly, and the eccentric shaft and the transmission collar are both disposed in the mounting sleeve. The eccentric transmission shaft is drivingly connected to the connecting shaft via a structural tube. A structural support sleeve is disposed between the structural tube and the housing, and a sealing sleeve is disposed between the central transmission shaft and the structural support sleeve.

A first water injection channel is formed between the mounting sleeve and the housing, a second water injection channel is formed between the structural support sleeve and the housing, and a third water injection channel is formed between the structural tube and the housing. The first, second, and third water injection channels are in sequential communication, and a water outlet of the third water injection channel is located at an end of the structural tube proximal to the grinding/milling cutter head.

According to another aspect of the present invention, a surgical reciprocating grinding instrument comprises a cutter head and a drive conversion assembly configured to drive the cutter head to perform a reciprocating rotational motion. The drive conversion assembly includes a transmission shaft, an input shaft, and an adapter. One end of the transmission shaft is connected to the cutter head, and a first eccentric post is fixed to the other end. The adapter includes a yoke and a conversion link. The first eccentric post is mounted to one end of the conversion link, and one prong of the yoke is connected to the other end of the conversion link. A first bearing is mounted in a fork opening of the yoke. A second eccentric post is fixed to one end of the input shaft, is eccentrically arranged relative to the input shaft, and is inserted into the first bearing. The yoke cooperates with the first bearing and the conversion link to convert a unidirectional rotational motion of the input shaft into a reciprocating rotational motion of the transmission shaft.

Preferably, the reciprocating grinding instrument further comprises a case, through which the transmission shaft extends. A pin is inserted into the case, and the yoke is rotatably mounted on the pin.

Insertion holes are formed at opposite ends of the conversion link. A connecting post is fixed to one prong of the yoke and is inserted into one of the insertion holes, and the first eccentric post is inserted into the other of the insertion holes.

The transmission shaft and the input shaft are coaxially arranged.

The reciprocating grinding instrument further comprises an interface housing and a housing sleeve. The input shaft extends through the interface housing. One end of the housing sleeve is fitted over the case, and the other end is fitted over the interface housing.

A second bearing is mounted in the interface housing and fitted over an end of the input shaft proximal to the second eccentric post.

An outer cutter tube is fitted over the transmission shaft, and an end of the outer cutter tube distal to the cutter head is inserted into the case.

According to another aspect of the present invention, a reciprocating drive structure for a cutting instrument is provided for connecting to and driving a cutting instrument assembly to reciprocally rotate. The reciprocating drive structure comprises:
an eccentric rocker rod fixedly connected to the cutting instrument assembly and configured to drive the cutting instrument assembly to reciprocally rotate, the eccentric rocker rod including a first connecting rod and a first eccentric rod that are fixedly connected with each other, wherein the first connecting rod is eccentrically arranged relative to the first eccentric rod;
an eccentric shaft drivingly connected to a drive motor, the eccentric shaft including a second connecting rod and a second eccentric rod that are fixedly connected with each other, wherein the second connecting rod is eccentrically arranged relative to the second eccentric rod and drivingly connected to a rotating shaft of the drive motor; and
a reciprocating conversion link, wherein both the eccentric rocker rod and the eccentric shaft are mounted to the reciprocating conversion link, with the first connecting rod and the second connecting rod located on opposite sides of the reciprocating conversion link, respectively. Unidirectional rotation of the eccentric shaft drives the reciprocating conversion link to rotate, thereby driving the eccentric rocker rod to reciprocally rotate.

Preferably, the second connecting rod is connected to a transmission shaft that is drivingly connected to the rotating shaft of the drive motor.

The reciprocating drive structure further comprises a cutter head seat. The first connecting rod extends through the cutter head seat and is capable of reciprocally rotating therein while maintaining a fixed axis. The first eccentric rod is located outside the cutter head seat.

A first support tube is fitted over an end of the first connecting rod proximal to the cutting instrument assembly, and one end of the first support tube is inserted into the cutter head seat.

A bearing housing is disposed outside the eccentric shaft. A first bearing is mounted on the second connecting rod and installed in the bearing housing.

According to another aspect of the present invention, a cutting instrument further comprises a water injection tube extending through the cutter head seat. A water outlet end of the water injection tube is located on an end face of the cutter head seat facing the cutting instrument assembly.

The cutting instrument further comprises an adapter sleeve assembly, a second support tube, and a spiral water cooling jacket. One end of the second support tube is connected to the cutter head seat, and the other end is connected to the adapter sleeve assembly. The spiral water cooling jacket is located inside the adapter sleeve assembly. A spiral channel is formed on an outer wall of the spiral water cooling jacket and communicates with the water injection tube which extends through the second support tube.

The adapter sleeve assembly includes an adapter tube and a case mounted on the adapter tube. The spiral water cooling jacket is located inside the case. The spiral channel on the outer wall of the spiral water cooling jacket and an inner wall of the case form part of a water injection channel. The transmission shaft extends through the adapter tube.

According to another aspect of the present invention, a medical reciprocating drill for orthopedic drilling comprises:
a drill assembly configured to perform a reciprocating rotational motion to drill into a bone tissue;
a drive assembly configured to drive the drill assembly to perform the reciprocating rotational motion, the drive assembly including a reciprocating conversion rod, a connecting sleeve, and an input rod, wherein the reciprocating conversion rod is connected to the drill assembly, one end of the connecting sleeve is fitted over the reciprocating conversion rod, the other end is fitted over the input rod, the input rod and the reciprocating conversion rod are eccentrically arranged, and rotation of the input rod drives the reciprocating conversion rod to perform the reciprocating rotational motion; and
a water injection channel configured to spray water onto a drilling site on the bone tissue, wherein an outer tube is fitted over the reciprocating conversion rod, and a gap between the outer tube and the reciprocating conversion rod forms the water injection channel.

Preferably, the drill assembly includes a connecting rod and a drill body fixedly connected to the connecting rod. The drill body includes a cone and a cylinder that are fixedly connected with each other.

Drill teeth are formed on side walls of both the cone and the cylinder. An end of the connecting rod proximal to the drill body is connected to the cylinder, and an end of the connecting rod distal to the drill body is inserted into the reciprocating conversion rod.

The reciprocating conversion rod includes a transmission rod and an eccentric post, wherein the eccentric post is eccentrically arranged relative to the transmission rod. An eccentric shaft is fixed to an end of the input rod proximal to the reciprocating conversion rod and is eccentrically arranged relative to the input rod. A connecting hole is formed at one end of the connecting sleeve, and a mounting hole is formed at the other end. The eccentric post extends through the connecting hole, and the eccentric shaft is mounted in the mounting hole. A bearing is mounted in the mounting hole and fitted over the eccentric shaft.

The reciprocating drill further comprises a front case, a spiral water cooling jacket, and a rear case. The outer tube and the reciprocating conversion rod both extend through the front case. A spiral channel is formed on an outer wall of the spiral water cooling jacket. A water inlet hole communicating with an interior is formed on the front case. A water outlet end of the spiral channel communicates with the water inlet hole, and the water inlet hole communicates with the gap between the outer tube and the reciprocating conversion rod. The rear case is fitted over the spiral water cooling jacket and connected to the front case.

A water passage groove is formed at an end of the outer tube distal to the drill assembly and communicates with an interior of the outer tube. The water inlet hole communicates with the water passage groove.

A sealing sleeve is fitted over an end of the transmission rod distal to the drill assembly, and a retaining sleeve is further fitted between the sealing sleeve and the transmission rod.

A water passage tube is fitted over an end of the transmission rod proximal to the drill assembly. One outer side wall of the water passage tube contacts an inner wall of the outer tube, and a channel communicating with an exterior is formed between the other outer side wall and the inner wall of the outer tube.

A circumferential slot is formed on an outer wall of the front case, and a snap ring is formed on the rear case. The snap ring is embedded in the circumferential slot to form a seal.

### Advantageous Effects

Compared with the prior art, the present invention provides the following advantageous effects:
1. By integrating the cutter head assembly and the drive assembly into a single product and powering it via an external drive member, the present invention achieves a compact overall structure and convenient maintenance. Furthermore, it may convert unidirectional motor rotation into reciprocating rotational motion, reciprocating swinging motion, or linear reciprocating motion of the working portion of the cutter head assembly.
2. By integrating the saw blade and the drive assembly into one product, the drive assembly may convert externally input unidirectional rotation into a linear reciprocating motion of the saw blade through cooperation of the eccentric shaft, the articulating connector, and the transmission rod, providing a compact structure. A water cooling channel is also integrated into the product and may communicate with an external water source to flush and cool the saw blade, thereby improving its service life.
3. The present invention converts the unidirectional rotation via the eccentric shaft and the connecting frame on the oscillating blade seat into reciprocating swinging of the oscillating blade about its hinge axis, thereby performing reciprocating cutting operations of the oscillating blade. By positioning the eccentric shaft close to the oscillating blade, the stability of the reciprocating motion of the oscillating blade may be improved.
4. The present invention converts the unidirectional rotation via the eccentric shaft and transmission swing rod into reciprocating rotation of the central transmission shaft, thereby driving the trephine to reciprocally rotate. External cooling water may be supplied to cool the trephine.
5. The present invention converts the unidirectional rotation via cooperation of the eccentric shaft, the transmission collar, and the eccentric transmission shaft into reciprocating rotation of the connecting shaft, thereby driving the grinding/milling cutter head to reciprocally rotate. A water injection channel is provided to flush and cool the grinding/milling cutter head, improving its service life. The concave central end-face design of the grinding/milling cutter head further improves cutting efficiency.
6. The present invention uses an external motor to rotate the input shaft unidirectionally, causing the second eccentric post to rotate eccentrically. In cooperation with the first bearing, the yoke swings back and forth circumferentially, driving the conversion link to swing back and forth. This in turn drives the transmission shaft to perform the reciprocating rotational motion via the first eccentric post, thereby driving the cutter head to grind a bone tissue. Reciprocating rotational motion ensures uniform force distribution, a clean and stable resection surface, smoother bone processing, improved grinding results, and accelerated wound recovery.
7. Water is injected into the spiral channel on the outer wall of the spiral water cooling jacket, flows into the water injection tube via the connection hole in the front case, and exits from the end face of the cutter head seat, thereby flushing and cooling the cutting instrument assembly.
8. An external water injection mechanism supplies water into the reciprocating drill, which then flows out through the outer tube. Specifically, the supplied water passes through the spiral channel, enters the water inlet hole, flows into the gap between the outer tube and the transmission rod through the water inlet hole and the water passage groove, and finally exits from the end of the outer tube proximal to the drill body, flushing and cooling the drilling site to improve drilling efficiency.
9. By means of the water injection channel integrated into the product, a coolant may absorb heat generated by the reciprocating conversion mechanism as it passes through the mechanism, cooling the mechanism and improving user experience.

### BRIEF DESCRIPTION OF THE DRAWINGS

Other features, objects, and advantages of the present invention will become more apparent upon reading the detailed description of non-limiting embodiments with reference to the following accompanying drawings:
FIG. 1 is a cross-sectional view schematically illustrating the overall structure of a reciprocating cutting instrument according to a first embodiment of the present invention;
FIG. 2 is a schematic view illustrating the overall structure of the reciprocating cutting instrument according to the first embodiment of the present invention;
FIG. 3 is an exploded view illustrating the components of the reciprocating cutting instrument according to the first embodiment of the present invention;
FIG. 4 is a cross-sectional view schematically illustrating the overall structure of a short-handle oscillating blade according to a second embodiment of the present invention;
FIG. 5 is a cross-sectional view schematically illustrating the overall structure of a long-handle oscillating blade according to the second embodiment of the present invention;
FIG. 6 is a schematic view illustrating the structure of the oscillating blade instrument according to the second embodiment of the present invention;
FIG. 7 is a schematic view illustrating the structure of the housing of the short-handle oscillating blade according to the second embodiment of the present invention;
FIG. 8 is a cross-sectional view schematically illustrating the overall structure of a reciprocating cutting instrument according to a third embodiment of the present invention;
FIG. 9 is a schematic view illustrating the overall structure of a trephine according to the third embodiment of the present invention;
FIG. 10 is an exploded view illustrating the components of the trephine according to the third embodiment of the present invention;
FIG. 11 is a cross-sectional view schematically illustrating the overall structure of a reciprocating cutting instrument according to a fourth embodiment of the present invention;
FIG. 12 is a schematic view illustrating the overall structure of a grinding/milling cutter instrument according to the fourth embodiment of the present invention;
FIG. 13 is an exploded view illustrating the components of the grinding/milling cutter instrument according to the fourth embodiment of the present invention;
FIG. 14 is a schematic view illustrating the structure of an eccentric shaft according to the fourth embodiment of the present invention;
FIG. 15 is a cross-sectional view of a surgical reciprocating grinding instrument according to a fifth embodiment of the present invention;
FIG. 16 is an enlarged view of portion A in FIG. 15 according to the fifth embodiment of the present invention;
FIG. 17 is a schematic view illustrating the structure of the surgical reciprocating grinding instrument according to the fifth embodiment of the present invention;
FIG. 18 is an enlarged schematic view illustrating the structure of an adapter, a transmission shaft, and an input shaft according to the fifth embodiment of the present invention;
FIG. 19 is a schematic view illustrating the assembly structure of the adapter and the transmission shaft according to the fifth embodiment of the present invention;
FIG. 20 is a cross-sectional view of a reciprocating drive structure for a cutting instrument according to a sixth embodiment of the present invention;
FIG. 21 is a schematic view illustrating the structures of an eccentric rocker rod, a reciprocating conversion link, and an eccentric shaft according to the sixth embodiment of the present invention;
FIG. 22 is a first cross-sectional view of a cutting instrument according to the sixth embodiment of the present invention;
FIG. 23 is an enlarged view of portion A in FIG. 22 according to the sixth embodiment of the present invention;
FIG. 24 is a schematic view illustrating the structure of the cutting instrument according to the sixth embodiment of the present invention;
FIG. 25 is a second cross-sectional view of the cutting instrument according to the sixth embodiment of the present invention;
FIG. 26 is an exploded view schematically illustrating a medical reciprocating drill for orthopedic drilling according to a seventh embodiment of the present invention;
FIG. 27 is a schematic view illustrating the structure of the medical reciprocating drill for orthopedic drilling according to the seventh embodiment of the present invention;
FIG. 28 is a cross-sectional view illustrating the medical reciprocating drill for orthopedic drilling according to the seventh embodiment of the present invention;
FIG. 29 is an enlarged schematic view of portion A in FIG. 28 according to the seventh embodiment of the present invention; and
FIG. 30 is an enlarged schematic view illustrating the structure of a drill assembly according to the seventh embodiment of the present invention.

Description of Reference Numerals: 1001 Housing, 1002 Eccentric Shaft, 1003 Articulating Connector, 1004 Transmission Rod, 1005 Connecting Frame, 1006 swing rod, 1007 Saw Blade, 1008 Cutter head Seat, 1009 Connecting Rod, 1010 Mounting Sleeve, 1011 Support Sleeve, 1012 Sealing Sleeve, 1013 Connecting Sleeve, 1014 Installation Cylinder, 1015 Sealing Ring, 2001 Housing, 2002 Oscillating Blade, 2003 Oscillating Blade Seat, 2004 Eccentric Shaft, 2005 Connecting Frame, 2006 Rotating Shaft, 2007 Transmission Shaft, 2008 Mounting Sleeve, 2009 Structural Support Sleeve, 2010 Sealing Sleeve, 2011 Connecting Sleeve, 2012 Installation Cylinder, 2013 Sealing Ring, 3001 Housing, 3002 Trephine, 3003 Eccentric Shaft, 3004 Transmission swing rod, 3005 Central Transmission Shaft, 3006 Mounting Sleeve, 3007 Installation Cylinder, 3008 Sealing Ring, 3009 Structural Support Sleeve, 3010 Sealing Sleeve, 3011 Fail-safe Sleeve, 3012 Weight-reduction Window, 4001 Housing, 4002 Eccentric Shaft, 4003 Transmission Collar, 4004 Eccentric Transmission Shaft, 4005 Connecting Shaft, 4006 Grinding/Milling Cutter Head, 4007 Mounting Sleeve, 4008 Installation Cylinder, 4009 Sealing Ring, 4010 Structural Tube, 4011 Structural Support Sleeve, 4012 Sealing Sleeve, 4013 Connecting Arcuate Surface, 5100 Cutter Head, 5110 Handle, 5210 Transmission Shaft, 5211 First Eccentric Post, 5212 Connecting Hole, 5220 Input Shaft, 5221 Second Eccentric Post, 5230 Yoke, 5231 Mounting Hole, 5232 Connecting Post, 5240 Conversion Link, 5241 Insertion Hole, 5250 First Bearing, 5260 Pin, 5270 Second Bearing, 5310 Case, 5320 Interface Housing, 5330 Housing Sleeve, 5410 Outer Cutter Tube, 5420 Support Tube, 6100 Eccentric rocker rod, 6110 First Connecting Rod, 6120 First Eccentric Rod, 6200 Eccentric Shaft, 6210 Second Connecting Rod, 6220 Second Eccentric Rod, 6230 Transmission Shaft, 6300 Reciprocating Conversion Link, 6310 Insertion Hole, 6400 Input Shaft, 6510 Cutter head Seat, 6520 First Support Tube, 6530 Bearing Housing, 6540 First Bearing, 6600 Water Injection Tube, 6710 Adapter Sleeve Assembly, 6711 Adapter Tube, 6712 Front Housing, 67120 Connection Hole, 6713 Rear Housing, 6720 Second Support Tube, 6730 Spiral Water Cooling Jacket, 6731 Spiral Channel, 6800 Second Bearing, 6001 Cutting Instrument Assembly, 7110 Connecting Rod, 7120 Drill Body, 7121 Cone, 7130 Drill Teeth, 7210 Reciprocating Conversion Rod, 7211 Transmission Rod, 7212 Eccentric Post, 7220 Connecting Sleeve, 7221 Connecting Hole, 7222 Mounting Hole, 7230 Input Rod, 7231 Eccentric Shaft, 7300 Water Injection Channel, 7400 Outer Tube, 7410 Water Passage Groove, 7420 Water Passage Tube, 7500 Bearing, 7610 Front Case, 7611 Water Inlet Hole, 7612 Circumferential Slot, 7620 Spiral Water Cooling Jacket, 7622 Spiral Channel, 7621 Water Injection Groove, 7630 Rear Case, 7631 Snap Ring, 7710 Sealing Sleeve, 7720 Retaining Sleeve, 7730 Bushing, 7800 Interface Housing, 7810 Bearing Spacer.

### DETAILED DESCRIPTIONS OF THE EMBODIMENTS

The present invention is described in detail below with reference to specific embodiments. The following embodiments are intended to assist those skilled in the art in further understanding the present invention, but are not intended to limit the invention in any form. It should be noted that persons of ordinary skill in the art may make several changes and modifications without departing from the concept of the present invention. Such changes and modifications all fall within the scope of protection of the present invention.

### First Embodiment

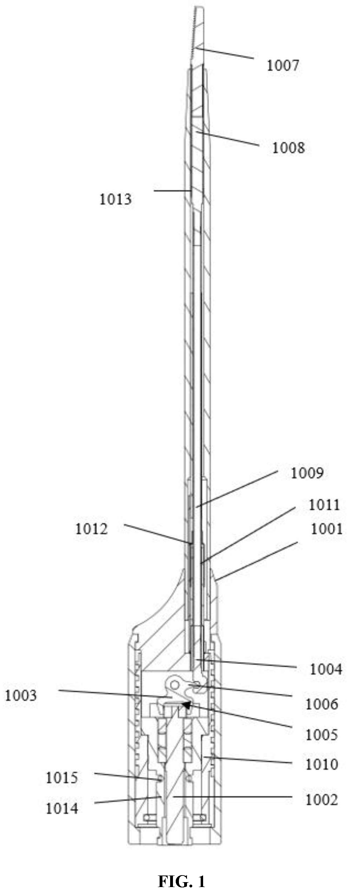

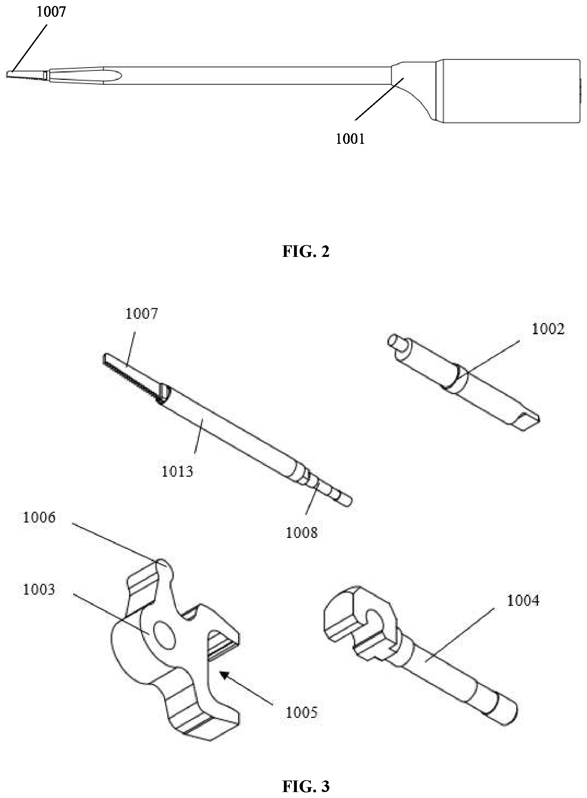

As shown in FIGS. 1 to 3, according to one aspect of the present invention, a reciprocating cutting instrument comprises a cutter head assembly and a drive assembly. The drive assembly is drivingly connected to the cutter head assembly and drives the cutter head assembly to perform a reciprocating motion. It should be noted that in the technical solution of the present application, the cutter head assembly and the drive assembly are integrated into a single product and driven by an external drive member, resulting in a compact overall structure and convenient maintenance. Furthermore, the technical solution of the present application is capable of converting unidirectional motor rotation into reciprocating rotational motion, reciprocating oscillating motion, or linear reciprocating motion of a working portion of the cutter head assembly.

In one feasible implementation, a reciprocating cutting instrument comprises a housing 1001, a cutter head assembly, and a drive assembly. The drive assembly includes an eccentric shaft 1002, an articulating connector 1003, and a transmission rod 1004. The eccentric shaft 1002, the articulating connector 1003, the transmission rod 1004, and the cutter head assembly are all disposed within the housing 1001, and a working end of the cutter head assembly extends out of the housing 1001. The articulating connector 1003 includes a connecting frame 1005 and a swing rod 1006, and is rotatably mounted in the housing 1001. One end of the eccentric shaft 1002 extends into the connecting frame 1005 and is rotatably engaged therewith. The swing rod 1006 is hinged to one end of the transmission rod 1004, and the other end of the transmission rod 1004 is drivingly connected to the cutter head assembly. Rotation of the eccentric shaft 1002 drives the articulating connector 1003 to reciprocally swing about its hinge axis, and the swing rod 1006 of the articulating connector 1003 drives the transmission rod 1004 to perform a linear reciprocating motion along an axis of the transmission rod 1004.

Specifically, the housing 1001 is composed of a case and an outer tube. One end of the outer tube extends into the case and is fixedly connected thereto. The working end of the cutter head assembly extends out of an end of the outer tube distal to the case. A mounting sleeve 1010 is disposed at an end of the housing 1001 distal to the cutter head assembly, and the eccentric shaft 1002 and the articulating connector 1003 are both disposed in the mounting sleeve 1010. An installation cylinder 1014 is fixedly disposed on a side of the mounting sleeve 1010 distal to the cutter head assembly. The eccentric shaft 1002 is rotatably mounted in the installation cylinder 1014 via a bearing, and an eccentric end of the eccentric shaft 1002 extends out of the installation cylinder 1014. A connector capable of being drivingly connected to an external motor is disposed at the other end of the eccentric shaft 1002. A first water injection channel is formed between the mounting sleeve 1010 and the housing 1001. A water inlet of the first water injection channel is located at an end of the mounting sleeve 1010 distal to the cutter head assembly. A sealing ring 1015 is embedded and mounted in an inner wall of the mounting sleeve 1010 at the water inlet, and a sealing ring 1015 is also disposed on an outer wall of the installation cylinder 1014. It should be noted that the technical solution of the present application does not limit the shape of the first water injection channel formed between the mounting sleeve 1010 and the housing 1001; the first water injection channel may be linear or spiral, and is preferably spiral. In actual use, the housing 1001 of an external drive device may be inserted between the mounting sleeve 1010 and the installation cylinder 1014 for connection, and an external water source may enter the interior of the first water injection channel through the water inlet of the first water injection channel. The two sealing rings 1015 can prevent water from entering the interior of the drive assembly, thereby protecting the drive assembly.

More specifically, the articulating connector 1003 includes a connecting frame 1005 and a swing rod 1006, with an angle formed therebetween, preferably 90 degrees. The axis of the transmission rod 1004 is parallel to the axis of the eccentric shaft 1002. The articulating connector 1003 is hinged to the mounting sleeve 1010 via a connecting shaft. The connecting frame 1005 is rotatably engaged with the eccentric shaft 1002. When the eccentric shaft 1002 is driven to rotate by an external drive, the connecting frame 1005 swings at a certain angle about the axis of the connecting shaft between the articulating connector 1003 and the mounting sleeve 1010, thereby driving the transmission rod 1004 to perform a reciprocating motion of a certain length along the axial direction of the transmission rod 1004 via the swing rod 1006.

The cutter head assembly includes a saw blade 1007, a cutter head seat 1008, and a connecting rod 1009. One end of the connecting rod 1009 is coaxially fixed to the transmission rod 1004, and the other end of the connecting rod 1009 is coaxially fixed to the cutter head seat 1008. The saw blade 1007 is fixedly mounted on an end of the cutter head seat 1008 distal to the connecting rod 1009 and extends out of the housing 1001. It should be emphasized that the saw blade 1007 in the technical solution of the present application has a uniform-thickness body, which may be formed by making the saw blade 1007 from a unitary plate material. An axial mounting groove is formed on the cutter head seat 1008, and one end of the saw blade 1007 is embedded and mounted in the axial mounting groove. The unitary saw blade 1007 has high structural strength.

Still more specifically, a second water injection channel is formed between the connecting rod 1009 and the housing 1001, and a third water injection channel is formed between the cutter head seat 1008 and the housing 1001. The first, second, and third water injection channels are in sequential fluid communication, enabling water injection from the exterior through the cutting instrument to a working site, thereby flushing and cooling the cutter head. Further preferably, a support sleeve 1011 is disposed between the housing 1001 and the connecting rod 1009, and a sealing sleeve 1012 is fitted over an end of the support sleeve 1011 distal to the articulating connector 1003. The sealing sleeve 1012 seals the junction between the support sleeve 1011 and the connecting rod 1009. Sealing the junction between the support sleeve 1011 and the connecting rod 1009 by the sealing sleeve 1012 can prevent cooling water from entering the drive assembly, providing waterproof protection for the drive assembly.

In one feasible implementation, with respect to the third water injection channel, a rectangular facet is formed on an outer surface of the cutter head seat 1008, and a connecting sleeve 1013 is disposed between the cutter head seat 1008 and a case. A third water injection channel is formed between the rectangular facet of the cutter head seat 1008 and the connecting sleeve 1013. A water outlet of the third water injection channel is located at an end of the connecting sleeve 1013 proximal to a working portion of the saw blade 1007.

In one feasible implementation, the cutter head seat 1008 is connected to the connecting rod 1009 as follows: the connecting rod 1009 is a hollow rod, and an end of the cutter head seat 1008 distal to the saw blade is a round rod, then the end of the cutter head seat 1008 distal to the saw blade extends into the interior of the connecting rod 1009 and is coaxially fixed thereto by adhesive bonding or welding.

In the present embodiment, by forming the saw blade and the drive assembly as a single product, the drive assembly may convert unidirectional rotation input from the exterior into a linear reciprocating motion of the saw blade through cooperation of the eccentric shaft 1002, the articulating connector 1003, and the transmission rod 1004, providing a compact structure. A water cooling channel is further integrated into the product, which may communicate with an external water source to flush and cool the saw blade, thereby improving the service life of the saw blade. It should be noted that the reciprocating conversion mechanism formed by cooperation of the eccentric shaft 1002, the articulating connector 1003, and the transmission rod 1004 in the product of the present embodiment generates high heat during a long-term operation. Cooling fluid in the water cooling channel may absorb heat generated by the reciprocating conversion mechanism as it passes through the mechanism, thereby improving user experience.

### Second Embodiment

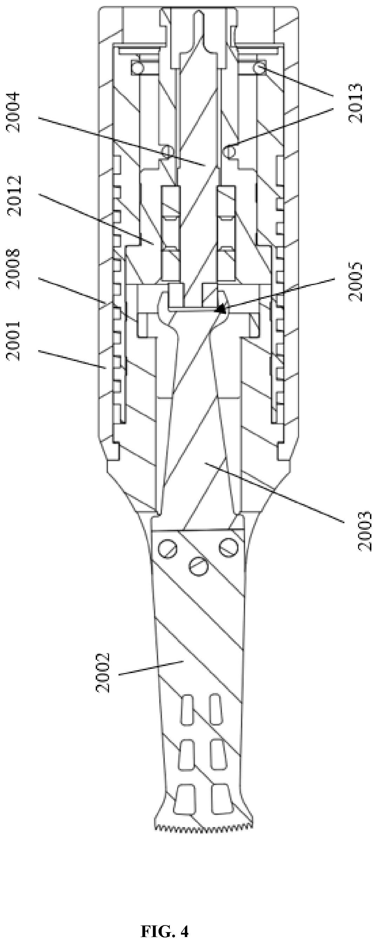

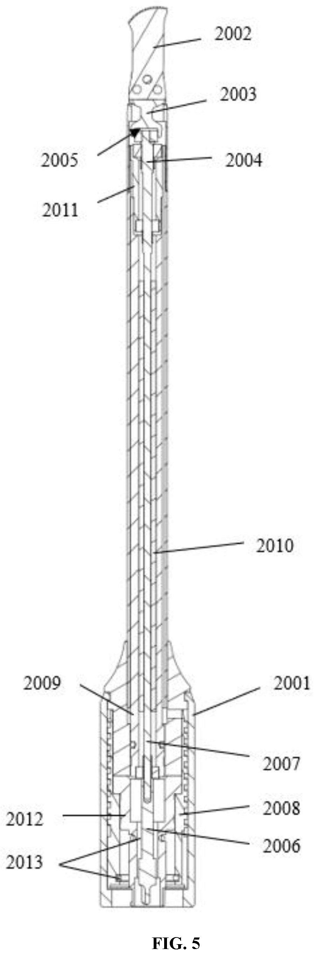

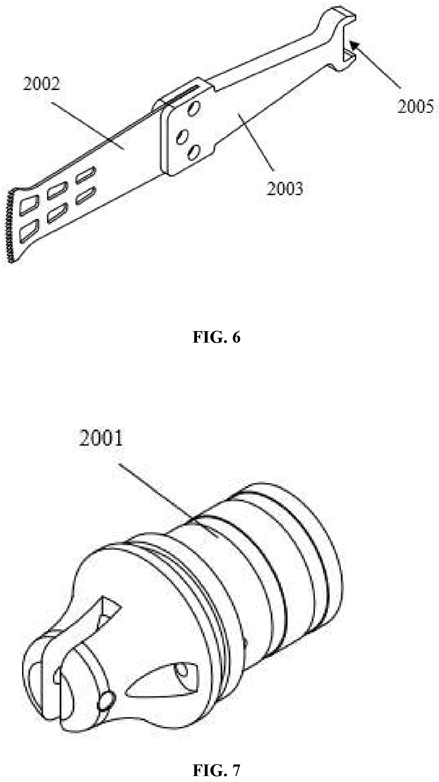

As shown in FIGS. 4 to 7, a reciprocating cutting instrument comprises a housing 2001, a cutter head assembly, and a drive assembly. The cutter head assembly includes an oscillating blade 2002 and an oscillating blade seat 2003. The oscillating blade 2002 is fixedly connected to the oscillating blade seat 2003, and the oscillating blade 2002 and/or the oscillating blade seat 2003 are hinged to the housing 2001. An end of the oscillating blade 2002 distal to the oscillating blade seat 2003 extends out of the housing 2001. The drive assembly includes an eccentric shaft 2004. A connecting frame 2005 is formed at an end of the oscillating blade seat 2003 distal to the oscillating blade 2002. The eccentric shaft 2004 extends into the connecting frame 2005 and is rotatably engaged therewith. Rotation of the eccentric shaft 2004 drives the end of the oscillating blade 2002 extending out of the housing 2001 to reciprocally swing about a hinge axis.

Different from the technical solution of the first embodiment, in the present embodiment, the drive assembly converts unidirectional rotation input from the exterior into a swinging motion of the oscillating blade 2002 within a certain angular range about its hinge axis with the housing 2001. The swing angle may be designed according to actual conditions.

Specifically, one end of the oscillating blade 2002 extends into a connecting groove of the oscillating blade seat 2003 and is fixedly connected thereto by a fixed connection means such as hinging, welding, or fasteners. The housing 2001 includes a case and an outer tube. One end of the outer tube extends into the case and is fixedly connected thereto. The oscillating blade 2002 and/or the oscillating blade seat 2003 are hinged to the outer tube via a rotating shaft 2006. A mounting sleeve 2008 is disposed at an end of the housing 2001 distal to the oscillating blade 2002. An installation cylinder 2012 is fixedly disposed on a side of the mounting sleeve 2008 distal to the cutter head assembly. The eccentric shaft 2004 is rotatably mounted in the installation cylinder 2012 via a bearing, and an eccentric end of the eccentric shaft 2004 extends out of the installation cylinder 2012. A connector capable of being drivingly connected to an external motor is disposed at the other end of the eccentric shaft 2004. A working portion of the oscillating blade 2002 is located at an end of the oscillating blade 2002 distal to the oscillating blade seat 2003. A connecting frame 2005 is disposed at an end of the oscillating blade seat 2003 distal to the oscillating blade 2002, and the connecting frame 2005 is rotatably engaged with the eccentric end of the eccentric shaft 2004. An external motor inputs unidirectional rotation to the eccentric shaft 2004, and rotation of the eccentric shaft 2004 drives the oscillating blade seat 2003 to reciprocally swing, thereby driving the oscillating blade head to reciprocally swing about the hinge axis.

Under different usage scenarios, different requirements are imposed on the length of the oscillating blade. For example, in a long-handle oscillating blade: the eccentric shaft 2004 is rotatably mounted at an end of the outer tube proximal to the cutter head assembly. The drive assembly further includes a rotating shaft 2006 and a transmission shaft 2007. The rotating shaft 2006 is coaxially fixed to the transmission shaft 2007, and an end of the transmission shaft 2007 distal to the rotating shaft 2006 is coaxially fixed to an end of the eccentric shaft 2004 distal to the oscillating blade seat 2003. A mounting sleeve 2008 is disposed at an end of the housing 2001 distal to the cutter head assembly, and the rotating shaft 2006 is disposed in the mounting sleeve 2008. An installation cylinder 2012 is fixedly disposed on a side of the mounting sleeve 2008 distal to the cutter head assembly. The rotating shaft 2006 is rotatably mounted in the installation cylinder 2012 via a bearing. A structural support sleeve 2009 is disposed between the transmission shaft 2007 and the housing 2001, and a sealing sleeve 2010 is disposed between the transmission shaft 2007 and the structural support sleeve 2009. A connecting sleeve 2011 is connected to an end of the structural support sleeve 2009 distal to the rotating shaft 2006. The eccentric shaft 2004 is rotatably disposed in the connecting sleeve 2011 via a bearing. Disposing the eccentric shaft 2004 close to the cutter head assembly may improve the stability of motion of the oscillating blade 2002.

In a preferred implementation, a water cooling channel is further integrated into a long-handle oscillating blade product. Water injection channels in fluid communication are formed between the mounting sleeve 2008, the structural support sleeve 2009, the connecting sleeve 2011, and the housing 2001, respectively. Openings of the water injection channels are located at an end of the connecting sleeve 2011 proximal to the cutter head assembly. The sealing sleeve 2010 disposed between the transmission shaft 2007 and the structural support sleeve 2009 may prevent cooling water from entering the drive assembly, providing waterproof protection for the drive assembly. A water inlet of the water injection channel is located at an end of the mounting sleeve 2008 distal to the cutter head assembly. A sealing ring 2013 is embedded and mounted in an inner wall of the mounting sleeve 2008 at the water inlet, and a sealing ring 2013 is also disposed on an outer wall of the installation cylinder 2012. It should be noted that the technical solution of the present application does not limit the shape of the water injection channel formed between the mounting sleeve 2008 and the housing 2001; the water injection channel may be linear or spiral, and is preferably spiral. In actual use, the housing 2001 of an external drive device may be inserted between the mounting sleeve 2008 and the installation cylinder 2012 for connection, and an external water source may enter the interior of the water injection channel through the water inlet. The two sealing rings 2013 can prevent water from entering the interior of the drive assembly, thereby protecting the drive assembly.

In a preferred implementation, in a short-handle oscillating blade, the water cooling channel is disposed between the mounting sleeve 2008 and the housing 2001, and a water outlet of the water cooling channel may be directly formed on the outer tube.

In the present embodiment, unidirectional drive of the motor is converted into reciprocating swinging of the oscillating blade 2002 about the hinge axis via the eccentric shaft 2004 and the connecting frame 2005 on the oscillating blade seat 2003, thereby implementing reciprocating cutting operations of the oscillating blade 2002. Furthermore, in the present application, disposing the eccentric shaft 2004 close to the oscillating blade 2002 may improve the stability of the reciprocating motion of the oscillating blade 2002. It should be noted that the reciprocating conversion mechanism formed by cooperation of the eccentric shaft 2004 and the oscillating blade seat 2003 in the product of the present embodiment generates high heat during a long-term operation. Cooling fluid in the water cooling channel may absorb heat generated by the reciprocating conversion mechanism as it passes through the mechanism, thereby improving user experience.

### Third Embodiment

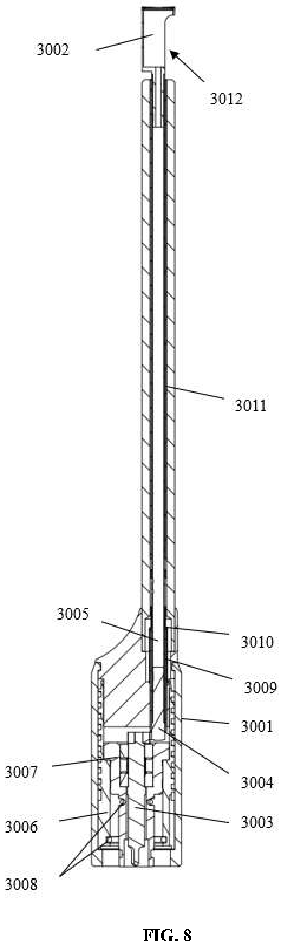

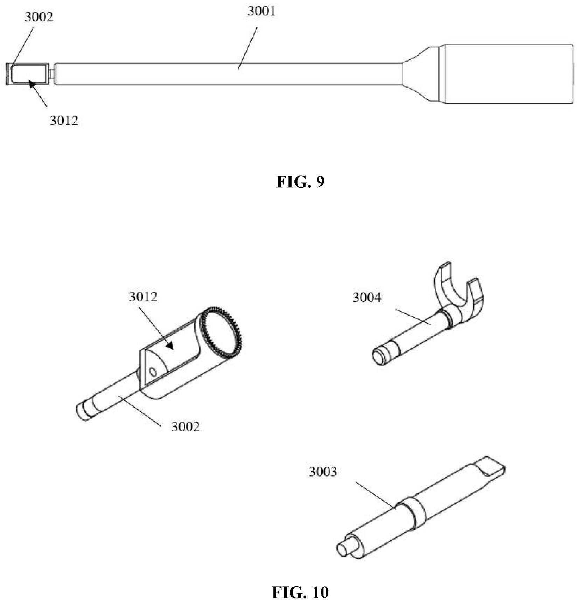

As shown in FIGS. 8 to 10, a reciprocating cutting instrument comprises a housing 3001, a cutter head assembly, and a drive assembly. The cutter head assembly includes a trephine 3002. The drive assembly includes an eccentric shaft 3003, a transmission swing rod 3004, and a central transmission shaft 3005. The transmission swing rod 3004 includes a connecting frame and a transmission rod, with the connecting frame located at one axial end of the transmission rod. The eccentric shaft 3003 extends into the connecting frame and is rotatably engaged therewith. An end of the transmission rod distal to the connecting frame is coaxially fixed to one end of the central transmission shaft 3005. The other end of the central transmission shaft 3005 is fixedly connected to the trephine 3002, and a working portion of the trephine 3002 extends out of the housing 3001. Rotation of the eccentric shaft 3003 drives the transmission swing rod 3004 to reciprocally swing, and the transmission swing rod 3004 drives the trephine 3002 to reciprocally swing via the central transmission shaft 3005.

Specifically, the housing 3001 is composed of a case and an outer tube. One end of the outer tube extends into the case and is fixedly connected thereto. The working end of the cutter head assembly extends out of an end of the outer tube distal to the case. A mounting sleeve 3006 is disposed at an end of the housing 3001 distal to the cutter head assembly, and the eccentric shaft 3003 and the transmission swing rod 3004 are both disposed in the mounting sleeve 3006. An installation cylinder 3007 is fixedly disposed on a side of the mounting sleeve 3006 distal to the cutter head assembly. The eccentric shaft 3003 is rotatably mounted in the installation cylinder 3007 via a bearing, and an eccentric end of the eccentric shaft 3003 extends out of the installation cylinder 3007. A connector capable of being drivingly connected to an external motor is disposed at the other end of the eccentric shaft 3003.

A water injection channel is formed between the mounting sleeve 3006 and the housing 3001. A water inlet of the water injection channel is located at an end of the mounting sleeve 3006 distal to the cutter head assembly. A sealing ring 3008 is embedded and mounted in an inner wall of the mounting sleeve 3006 at the water inlet, and a sealing ring 3008 is also disposed on an outer wall of the installation cylinder 3007. It should be noted that the technical solution of the present application does not limit the shape of the water injection channel formed between the mounting sleeve 3006 and the housing 3001; the water injection channel may be linear or spiral, and is preferably spiral. In actual use, the housing 3001 of an external drive device may be inserted between the mounting sleeve 3006 and the installation cylinder 3007 for connection, and an external water source may enter the interior of the water injection channel through the water inlet. The two sealing rings 3008 can prevent water from entering the interior of the drive assembly, thereby protecting the drive assembly.

A mounting sleeve 3006 is disposed at an end of the housing 3001 distal to the cutter head assembly, and the eccentric shaft 3003 is disposed in the mounting sleeve 3006. A structural support sleeve 3009 is disposed between the central transmission shaft 3005 and the housing 3001, and a sealing sleeve 3010 is disposed between the central transmission shaft 3005 and the structural support sleeve 3009. The sealing sleeve 3010 seals the junction between the central transmission shaft 3005 and the structural support sleeve 3009, providing waterproof protection for the drive assembly. A fail-safe sleeve 3011 is disposed between the central transmission shaft 3005 and the housing 3001. The fail-safe sleeve 3011 may prevent the trephine 3002 from falling off in the event of failure between the trephine 3002 and the central transmission shaft 3005, thereby improving safety in use.

A water injection channel is formed between the mounting sleeve 3006 and the housing 3001. A through hole communicating an inner cavity of the central transmission shaft 3005 with the water injection channel is formed on the central transmission shaft 3005. A water injection hole is formed inside the trephine 3002 and communicates with the inner cavity of the central transmission shaft 3005. This enables water injection from the exterior through the interior of the product to flush and cool the working environment, thereby improving the service life of the trephine 3002.

In a preferred implementation, the cutter head of the trephine 3002 is a hollow ring, and a weight-reduction window 3012 is formed in a body portion of the trephine 3002, improving the stability of motion of the trephine 3002.

In the technical solution of the present embodiment, unidirectional rotation input by the motor is converted into reciprocating rotation of the central transmission shaft 3005 via the eccentric shaft 3003 and the transmission swing rod 3004, thereby implementing reciprocating rotation of the trephine 3002. Furthermore, the trephine 3002 may be cooled by supplying external cooling water. It should be noted that the reciprocating conversion mechanism formed by cooperation of the eccentric shaft 3003, transmission swing rod 3004, and central transmission shaft 3005 in the product of the present embodiment generates high heat during a long-term operation. Cooling fluid in the water cooling channel may absorb heat generated by the reciprocating conversion mechanism as it passes through the mechanism, thereby improving user experience.

### Fourth Embodiment

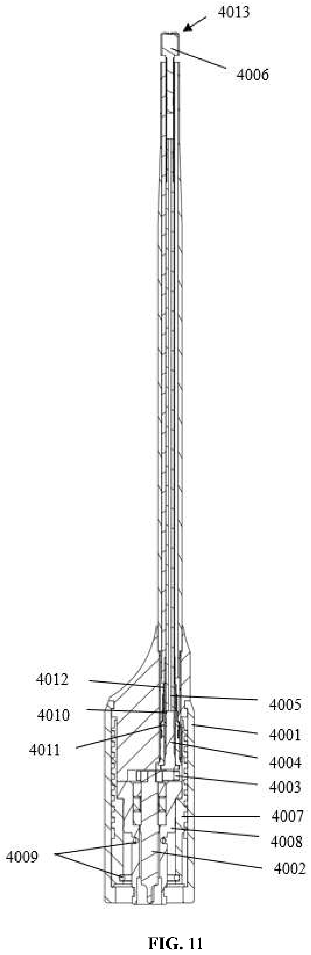

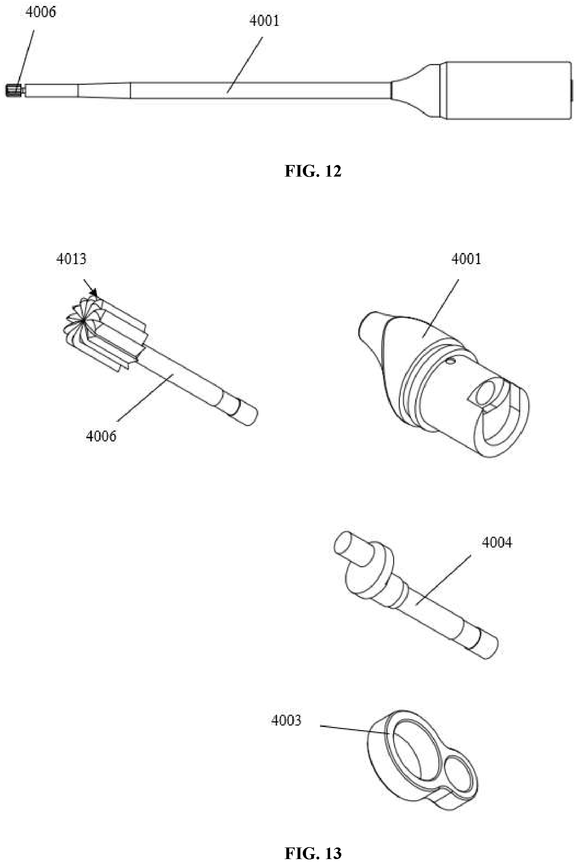

As shown in FIGS. 11 to 13, a reciprocating cutting instrument comprises a housing 4001, a cutter head assembly, and a drive assembly. The drive assembly includes an eccentric shaft 4002, a transmission collar 4003, an eccentric transmission shaft 4004, and a connecting shaft 4005. The cutter head assembly includes a grinding/milling cutter head 4006. The transmission collar 4003 includes a first ring portion and a second ring portion. The first ring portion is fitted over the eccentric shaft 4002 and rotatably engaged therewith. The eccentric transmission shaft 4004 extends into the second ring portion and is rotatably engaged therewith. An end of the eccentric transmission shaft 4004 distal to the transmission collar 4003 is drivingly connected to the connecting shaft 4005. An end of the connecting shaft 4005 distal to the transmission shaft is drivingly connected to the grinding/milling cutter head 4006. Rotation of the eccentric shaft 4002 drives the eccentric transmission shaft 4004 to rotate via the transmission collar 4003, thereby driving the grinding/milling cutter head 4006 to reciprocally rotate via the connecting shaft 4005.

Specifically, the housing 4001 is composed of a case and an outer tube. One end of the outer tube extends into the case and is fixedly connected thereto. The working end of the cutter head assembly extends out of an end of the outer tube distal to the case. A mounting sleeve 4007 is disposed at an end of the housing 4001 distal to the cutter head assembly, and the eccentric shaft 4002 and the transmission collar 4003 are both disposed in the mounting sleeve 4007. An installation cylinder 4008 is fixedly disposed on a side of the mounting sleeve 4007 distal to the cutter head assembly. The eccentric shaft 4002 is rotatably mounted in the installation cylinder 4008 via a bearing, and an eccentric end of the eccentric shaft 4002 extends out of the installation cylinder 4008. A connector capable of being drivingly connected to an external motor is disposed at the other end of the eccentric shaft 4002. A first water injection channel is formed between the mounting sleeve 4007 and the housing 4001. A water inlet of the first water injection channel is located at an end of the mounting sleeve 4007 distal to the cutter head assembly. A sealing ring 4009 is embedded and mounted in an inner wall of the mounting sleeve 4007 at the water inlet, and a sealing ring 4009 is also disposed on an outer wall of the installation cylinder 4008. It should be noted that the technical solution of the present application does not limit the shape of the first water injection channel formed between the mounting sleeve 4007 and the housing 4001; the first water injection channel may be linear or spiral, and is preferably spiral. In actual use, the housing 4001 of an external drive device may be inserted between the mounting sleeve 4007 and the installation cylinder 4008 for connection, and an external water source may enter the interior of the first water injection channel through the water inlet. The two sealing rings 4009 can prevent water from entering the interior of the drive assembly, thereby protecting the drive assembly.

More specifically, a mounting sleeve 4007 is disposed at an end of the housing 4001 distal to the cutter head assembly, and the eccentric shaft 4002 and the transmission collar 4003 are both disposed in the mounting sleeve 4007. The eccentric transmission shaft 4004 is drivingly connected to the connecting shaft 4005 via a structural tube 4010. A structural support sleeve 4011 is disposed between the structural tube 4010 and the housing 4001, and a sealing sleeve 4012 is disposed between the central transmission shaft and the structural support sleeve 4011. The sealing sleeve 4012 seals the junction between the central transmission shaft and the structural support sleeve 4011, implementing sealed protection for the drive assembly.

A first water injection channel is formed between the mounting sleeve 4007 and the housing 4001, a second water injection channel is formed between the structural support sleeve 4011 and the housing 4001, and a third water injection channel is formed between the structural tube 4010 and the housing 4001. The first, second, and third water injection channels are in sequential fluid communication, and a water outlet of the third water injection channel is located at an end of the structural tube 4010 proximal to the grinding/milling cutter head 4006. This enables water injection from the exterior and water passage through the interior of the product to flush and cool the grinding/milling cutter head 4006.

In a preferred implementation, grinding teeth are formed on both an end face and a side face of the grinding/milling cutter head 4006, and a connecting arcuate surface 4013 is formed between the grinding teeth on the end face and the grinding teeth on the side face of the grinding/milling cutter head 4006. The connecting arcuate surface 4013 may protect normal tissue of a patient during cutting, reducing accidental cuts. A central portion of the end face of the grinding/milling cutter head 4006 is concave, improving the cutting effect of the grinding/milling cutter head 4006.

In the present implementation, unidirectional rotation input by an external motor is converted into reciprocating rotation of the connecting shaft 4005 through cooperation of the eccentric shaft 4002, the transmission collar 4003, and the eccentric transmission shaft 4004, thereby driving the grinding/milling cutter head 4006 to reciprocally rotate. A water injection channel is further provided to flush and cool the grinding/milling cutter head 4006, improving its service life. Furthermore, the structural design of the grinding/milling cutter head 4006 with a concave central portion of the end face improves the cutting effect. It should be noted that the reciprocating conversion mechanism formed by cooperation of the eccentric shaft 4002, the transmission collar 4003, the eccentric transmission shaft 4004, and the connecting shaft 4005 in the product of the present embodiment generates high heat during a long-term operation. Cooling fluid in the water cooling channel may absorb heat generated by the reciprocating conversion mechanism as it passes through the mechanism, thereby improving user experience.

### Fifth Embodiment

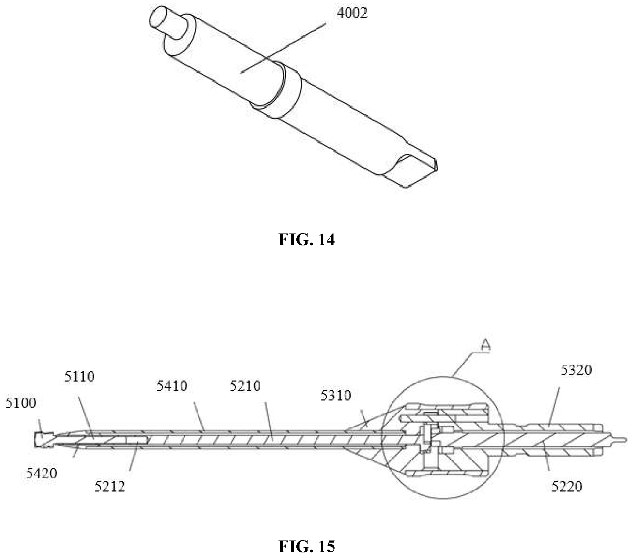

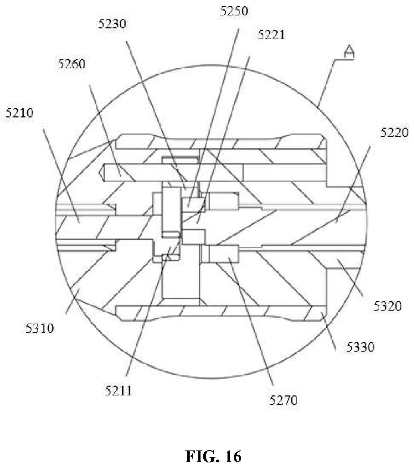

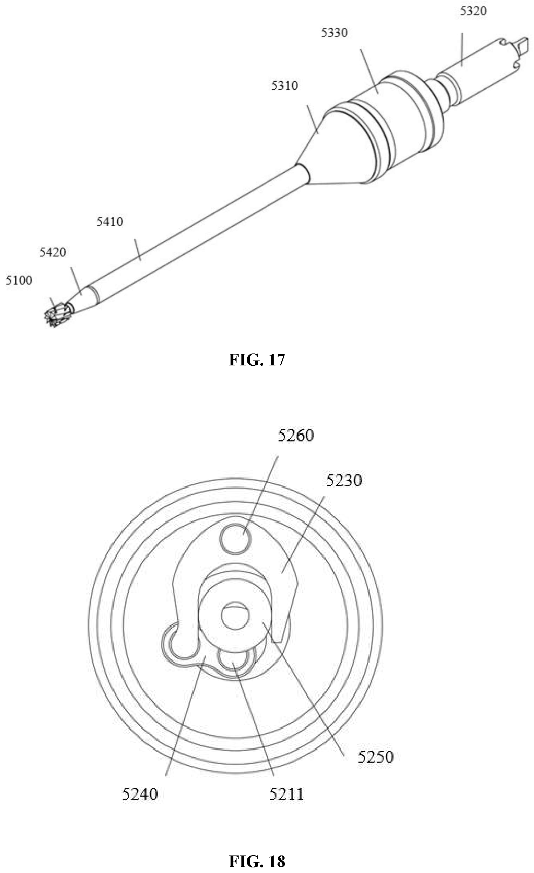

With reference to FIGS. 15 to 19, the present application provides a surgical reciprocating grinding instrument. The reciprocating grinding instrument in the present embodiment corresponds to the reciprocating cutting instrument in the first embodiment, and comprises a cutter head 5100 and a drive conversion assembly configured to drive the cutter head 5100 to perform a reciprocating rotational motion. The drive conversion assembly includes a transmission shaft 5210, an input shaft 5220, and an adapter. One end of the transmission shaft 5210 is connected to the cutter head 5100, and a first eccentric post 5211 is fixed to the other end. The adapter includes a yoke 5230 and a conversion link 5240. The first eccentric post 5211 is mounted to one end of the conversion link 5240, and one prong of the yoke 5230 is connected to the other end of the conversion link 5240. A first bearing 5250 is mounted in a fork opening of the yoke 5230. A second eccentric post 5221 is fixed to one end of the input shaft 5220, is eccentrically arranged relative to the input shaft 5220, and is inserted into the first bearing 5250. The yoke 5230 cooperates with the first bearing 5250 and the conversion link 5240 to convert a unidirectional rotational motion of the input shaft 5220 into the reciprocating rotational motion of the transmission shaft 5210.

In the surgical reciprocating grinding instrument adopting the above technical solution, an end of the input shaft 5220 distal to the second eccentric post 5221 is connected to a rotating shaft of an external motor. When the external motor is started, the input shaft 5220 is driven by the external motor to rotate unidirectionally, causing the second eccentric post 5221 to rotate eccentrically. In cooperation with the first bearing 5250, the yoke 5230 swings back and forth in the circumferential direction, driving the conversion link 5240 to swing back and forth. Consequently, under the action of the first eccentric post 5211, the transmission shaft 5210 is driven to perform a reciprocating rotational motion, thereby driving the cutter head 5100 to perform the reciprocating rotational motion to grind a bone tissue. Grinding the bone tissue with the reciprocating rotational motion facilitates uniform force distribution, provides a neat and stable grinding wound surface, ensures smoother processing of the bone tissue, effectively improves the grinding effect, and promotes wound recovery. In the present utility model, the reciprocating rotational motion means that the transmission shaft 5210 or the cutter head 5100 rotates a certain angle in a forward direction, then rotates a certain angle in a reverse direction, then rotates a certain angle in the forward direction again, and so on in a reciprocating cycle.

In the present embodiment, the cutter head 5100 has a handle 5110, and an end of the transmission shaft 5210 proximal to the cutter head 5100 has a connecting hole 5212. The handle 5110 is fixedly inserted into the connecting hole 5212 to implement a fixed connection between the transmission shaft 5210 and the cutter head 5100.

In the present embodiment, the reciprocating grinding instrument further comprises a case 5310, through which the transmission shaft 5210 extends. A pin 5260 is inserted into the case 5310, and the yoke 5230 is rotatably mounted on the pin 5260. Specifically, a top portion of the fork opening of the yoke 5230 has a mounting hole 5231, and the pin 5260 extends through the mounting hole 5231 to implement rotatable mounting of the yoke 5230 on the pin 5260.

In the present embodiment, insertion holes 5241 are formed at opposite ends of the conversion link 5240. A connecting post 5232 is fixed to one prong of the yoke 5230 and is inserted into one insertion hole 5241, and the first eccentric post 5211 is inserted into the other insertion hole 5241. This ensures that when the yoke 5230 swings back and forth in the circumferential direction, it can simultaneously drive the conversion link 5240 to swing back and forth, thereby driving the transmission shaft 5210 to perform a reciprocating rotational motion under the action of the first eccentric post 5211.

In the present embodiment, the transmission shaft 5210 and the input shaft 5220 are coaxially arranged.

In the present embodiment, the reciprocating grinding instrument further comprises an interface housing 5320 and a housing sleeve 5330. The input shaft 5220 extends through the interface housing 5320. One end of the housing sleeve 5330 is fitted over the case 5310, and the other end is fitted over the interface housing 5320. To make rotation of the input shaft 5220 more stable, a second bearing 5270 is mounted in the interface housing 5320 and fitted over an end of the input shaft 5220 proximal to the second eccentric post 5221.

In the present embodiment, to enhance rigidity and strength of the transmission shaft 5210 and protect the transmission shaft 5210, an outer cutter tube 5410 is fitted over the transmission shaft 5210. An end of the outer cutter tube 5410 distal to the cutter head 5100 is inserted into the case 5310. A support tube 5420 is further fitted over an end of the transmission shaft 5210 proximal to the cutter head 5100. An end of the support tube 5420 distal to the cutter head 5100 is inserted into the outer cutter tube 5410.

An end of the input shaft 5220 distal to the second eccentric post 5221 is connected to the rotating shaft of the external motor. When the external motor is started, the input shaft 5220 performs a unidirectional rotational motion, driving the second eccentric post 5221 to rotate eccentrically. The second eccentric post 5221 is inserted into the first bearing 5250, causing the first bearing 5250 to slide in the fork opening of the yoke 5230, driving the yoke 5230 to swing left and right in the circumferential direction. One prong of the yoke 5230 is connected to the conversion link 5240, driving the conversion link 5240 to reciprocally swing. The first eccentric post 5211 is inserted into the conversion link 5240, causing the transmission shaft 5210 to perform a reciprocating rotational motion and transmit the motion to the cutter head 5100, thereby causing the cutter head 5100 to also perform the reciprocating rotational motion to grind a bone tissue.

### Sixth Embodiment

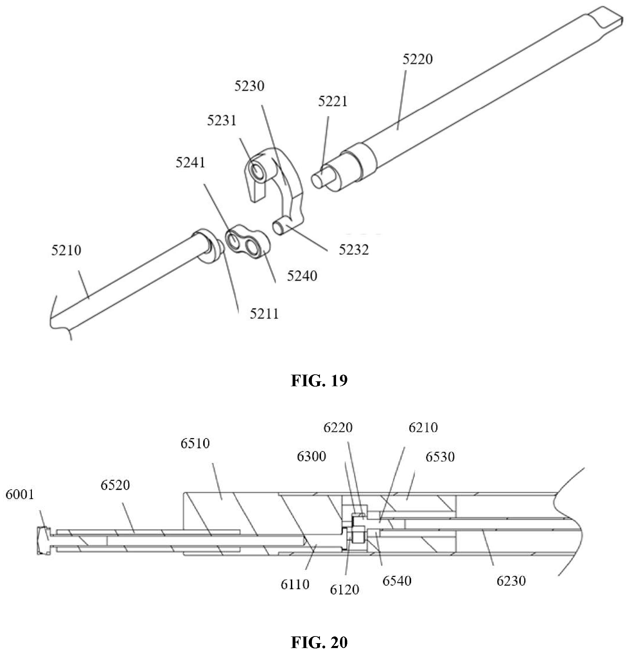

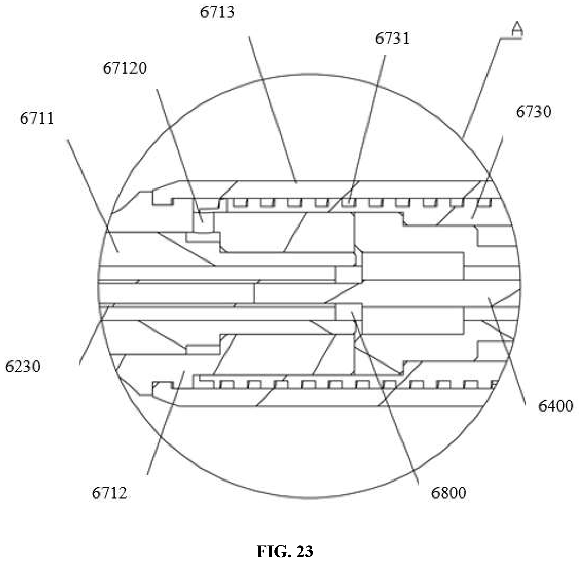

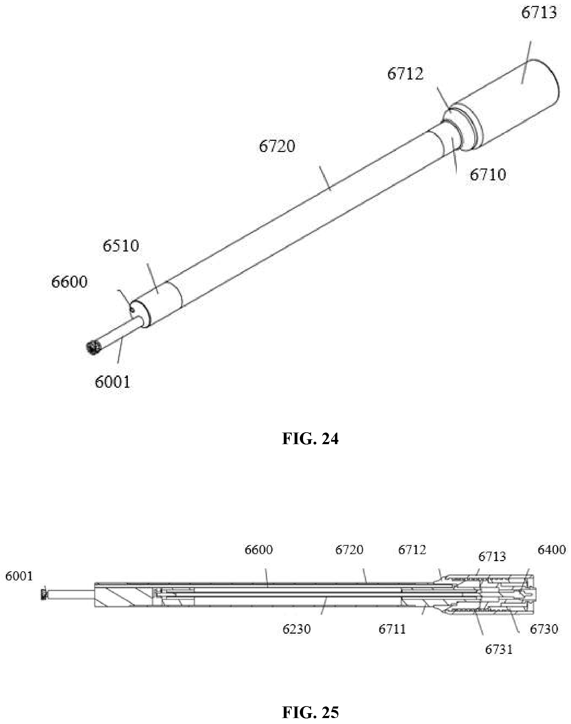

With reference to FIGS. 20 to 25, the present application provides a reciprocating drive structure for a cutting instrument. The reciprocating drive structure in the present embodiment corresponds to the reciprocating cutting instrument in the first embodiment, and is configured to connect to a cutting instrument assembly 6001 and drive the cutting instrument assembly 6001 to reciprocally rotate. The reciprocating drive structure of the present embodiment comprises an eccentric rocker rod 6100, an eccentric shaft 6200, and a reciprocating conversion link 6300. The eccentric rocker rod 6100 is fixedly connected to the cutting instrument assembly 6001 and drives the cutting instrument assembly 6001 to reciprocally rotate. The eccentric rocker rod 6100 includes a first connecting rod 6110 and a first eccentric rod 6120 that are fixedly connected with each other, wherein the first connecting rod 6110 is eccentrically arranged relative to the first eccentric rod 6120. The eccentric shaft 6200 is drivingly connected to a drive motor. The eccentric shaft 6200 includes a second connecting rod 6210 and a second eccentric rod 6220 that are fixedly connected with each other, wherein the second connecting rod 6210 is eccentrically arranged relative to the second eccentric rod 6220 and is drivingly connected to a rotating shaft of the drive motor. Both the eccentric rocker rod 6100 and the eccentric shaft 6200 are mounted to the reciprocating conversion link 6300, with the first connecting rod 6110 and the second connecting rod 6210 located on opposite sides of the reciprocating conversion link 6300, respectively. Unidirectional rotation of the eccentric shaft 6200 drives the reciprocating conversion link 6300 to rotate, thereby driving the eccentric rocker rod 6100 to reciprocally rotate.

In the present embodiment, the first connecting rod 6110 performs reciprocating rotation, driving the cutting instrument assembly 6001 connected to the first connecting rod 6110 to perform reciprocating rotation. Reciprocating rotation herein means that the cutting instrument assembly 6001 or the first connecting rod 6110 rotates a certain angle about its own axis, then rotates a certain angle in the opposite direction, then rotates a certain angle in the opposite direction again, and so on in a reciprocating cycle.

In the present embodiment, when the eccentric rocker rod 6100 and the eccentric shaft 6200 are both mounted to the reciprocating conversion link 6300, the axes of the first connecting rod 6110 and the second connecting rod 6210 are parallel to each other and non-coincident. The reciprocating conversion link 6300 simultaneously connected to both the eccentric rocker rod 6100 and the eccentric shaft 6200 has two insertion holes 6310. The two insertion holes 6310 are located at opposite ends of the reciprocating conversion link 6300, respectively, for insertion of the first eccentric rod 6120 and the second eccentric rod 6220. Inserting the first eccentric rod 6120 and the second eccentric rod 6220 into the corresponding insertion holes 6310, respectively, implements mounting of both the eccentric rocker rod 6100 and the eccentric shaft 6200 to the reciprocating conversion link 6300. Of course, other connection means may be selected for connecting the eccentric rocker rod 6100 and the eccentric shaft 6200 to the reciprocating conversion link 6300, as long as unidirectional rotation of the eccentric shaft 6200 can drive the reciprocating conversion link 6300 to rotate and drive the eccentric rocker rod 6100 to reciprocally rotate.

In the present embodiment, the reciprocating conversion link 6300 has a simple structure, and the connection and cooperation structure with the eccentric rocker rod 6100 and the eccentric shaft 6200 is also simple. The reciprocating conversion link 6300 may be mounted close to the cutting instrument assembly 6001, and the first connecting rod 6110 in the eccentric rocker rod 6100 is directly connected to the cutting instrument assembly 6001, so as to shorten the transmission distance of reciprocating rotation on the first connecting rod 6110, directly transmit reciprocating rotation on the first connecting rod 6110 to the cutting instrument assembly 6001, and more effectively implement reciprocating rotation of the cutting instrument assembly 6001.

In the present embodiment, the second connecting rod 6210 is connected to a transmission shaft 6230, which is drivingly connected to the rotating shaft of the drive motor. Specifically, the transmission shaft 6230 has a hollow tubular structure. The second connecting rod 6210 is fixedly inserted into one end of the transmission shaft 6230. An input shaft 6400 is connected to an end of the transmission shaft 6230 distal to the eccentric shaft 6200, and the input shaft 6400 is connected to the rotating shaft of the drive motor via a coupling. Rotation of the drive motor drives the input shaft 6400 to rotate, thereby driving the transmission shaft 6230 to rotate, and rotation of the transmission shaft 6230 drives the eccentric shaft 6200 to rotate.

In the present embodiment, the first connecting rod 6110 is fixedly connected to the cutting instrument assembly 6001. The present embodiment does not specifically limit the connection means between the first connecting rod 6110 and the cutting instrument assembly 6001, as long as the first connecting rod 6110 can be fixedly connected to the cutting instrument assembly 6001 and reciprocating rotation of the first connecting rod 6110 can drive the cutting instrument assembly 6001 to reciprocally rotate. A connecting rod of the cutting instrument assembly 6001 may be fixedly inserted into the first connecting rod 6110, or a connecting rod of the cutting instrument assembly 6001 may be fitted over the first connecting rod 6110.

In the present embodiment, the reciprocating drive structure further comprises a cutter head seat 6510. The first connecting rod 6110 extends through the cutter head seat 6510 and is capable of reciprocally rotating therein while maintaining a fixed axis. The first eccentric rod 6120 is located outside the cutter head seat 6510. Specifically, the cutter head seat 6510 has a through hole. The first connecting rod 6110 extends through the through hole, with one end of the first connecting rod 6110 extending out of the cutter head seat 6510 for connection to the cutting instrument assembly 6001, and the other end extending out of the cutter head seat 6510 for connection to the first eccentric rod 6120. A first support tube 6520 is fitted over an end of the first connecting rod 6110 proximal to the cutting instrument assembly 6001, and one end of the first support tube 6520 is inserted into the cutter head seat 6510.

In the present embodiment, a bearing housing 6530 is disposed outside the eccentric shaft 6200. A first bearing 6540 is mounted on the second connecting rod 6210 and installed in the bearing housing 6530.

Under rotation of the drive motor, the input shaft 6400 rotates to drive the transmission shaft 6230 to rotate, thereby driving the eccentric shaft 6200 to rotate and the second eccentric rod 6220 to perform eccentric rotation, which in turn drives the reciprocating conversion link 6300 to rotate. The reciprocating conversion link 6300 drives the first eccentric rod 6120 to rotate, thereby driving the first connecting rod 6110 to perform reciprocating rotation. The first connecting rod 6110 is connected to the cutting instrument assembly 6001, and the cutting instrument assembly 6001 performs reciprocating rotation synchronously with the first connecting rod 6110.

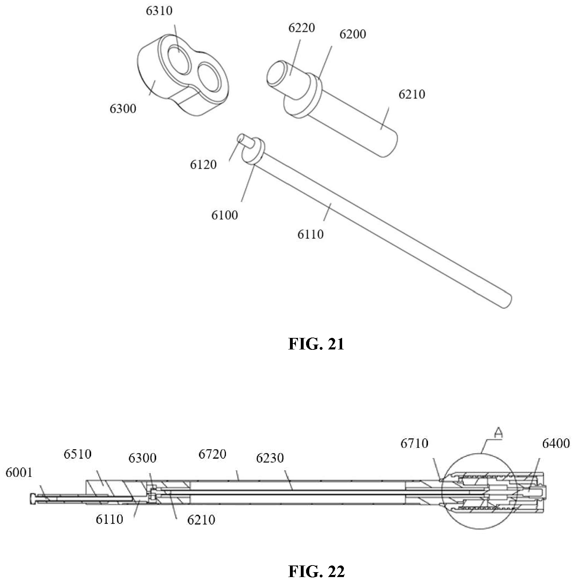

The cutting instrument according to the present embodiment is primarily an orthopedic instrument configured to perform drilling, grinding, and other operations on bone or tissue. Referring to FIGS. 22 to 25, the cutting instrument according to the present embodiment comprises the aforementioned reciprocating drive structure and the cutting instrument assembly 6001. The cutting instrument assembly 6001 is fixedly connected to the first connecting rod 6110 of the eccentric rocker rod 6100. The cutting instrument according to the present embodiment further comprises a water injection tube 6600 extending through the cutter head seat 6510. A water outlet end of the water injection tube 6600 is located on an end face of the cutter head seat 6510 facing the cutting instrument assembly 6001. Supplying water to the water injection tube 6600 flushes and cools the operating cutting instrument assembly 6001, which helps improve surgical efficiency to a certain extent.

The cutting instrument further comprises an adapter sleeve assembly 6710, a second support tube 6720, and a spiral water cooling jacket 6730. One end of the second support tube 6720 is connected to the cutter head seat 6510, and the other end is connected to the adapter sleeve assembly 6710. The spiral water cooling jacket 6730 is located inside the adapter sleeve assembly 6710. A spiral channel 6731 is formed on an outer wall of the spiral water cooling jacket 6730 and communicates with the water injection tube 6600, which extends through the second support tube 6720.

In the present embodiment, the adapter sleeve assembly 6710 comprises an adapter tube 6711 and a case mounted on the adapter tube 6711. One end of the second support tube 6720 is fitted over the adapter tube 6711, and the other end is fitted over the cutter head seat 6510. The bearing housing 6530 is installed inside the second support tube 6720. The spiral water cooling jacket 6730 is located inside the case. The spiral channel 6731 on the outer wall of the spiral water cooling jacket 6730 and an inner wall of the case together form part of a water injection channel. The transmission shaft 6230 extends through the adapter tube 6711. Specifically, the case comprises a front housing 6712 and a rear housing 6713. One end of the front housing 6712 is inserted into the rear housing 6713, and one end of the rear housing 6713 is snapped onto an outer wall of the front housing 6712. The rear housing 6713 is fitted over the spiral water cooling jacket 6730. The front housing 6712 defines a connection hole 67120 at a position corresponding to an outlet end of the spiral channel 6731. A water inlet end of the water injection tube 6600 is inserted into the connection hole 67120 and communicates with the spiral channel 6731. A second bearing 6800 is mounted on the input shaft 6400 and located inside the adapter tube 6711.

The cutting instrument according to the present embodiment may be configured as a grinding instrument by connecting a grinding-type cutting instrument assembly 6001 to the first connecting rod 6110, or as a drilling instrument by connecting a drilling-type cutting instrument assembly 6001 thereto. Under the interaction of the eccentric shaft 6200, the reciprocating conversion link 6300, and the eccentric rocker rod 6100, the cutting instrument according to the present embodiment may achieve reciprocating rotation of the first connecting rod 6110, thereby driving the cutting instrument assembly 6001 to reciprocally rotate to perform grinding, drilling, and other operations on bone and tissue. The first connecting rod 6110 is directly connected to the cutting instrument assembly 6001, and the reciprocating conversion link 6300 may be mounted close to the cutting instrument assembly 6001, which helps efficiently transmit reciprocating rotation to the cutting instrument assembly 6001. During surgery, the cutting instrument according to the present embodiment may inject water into the spiral channel 6731 on the outer wall of the spiral water cooling jacket 6730; the water then enters the water injection tube 6600 via the connection hole 67120 on the front housing 6712 and flows out from the end face of the cutter head seat 6510 through the injection tube 6600, thereby flushing and cooling the cutting instrument assembly 6001.

### Seventh Embodiment

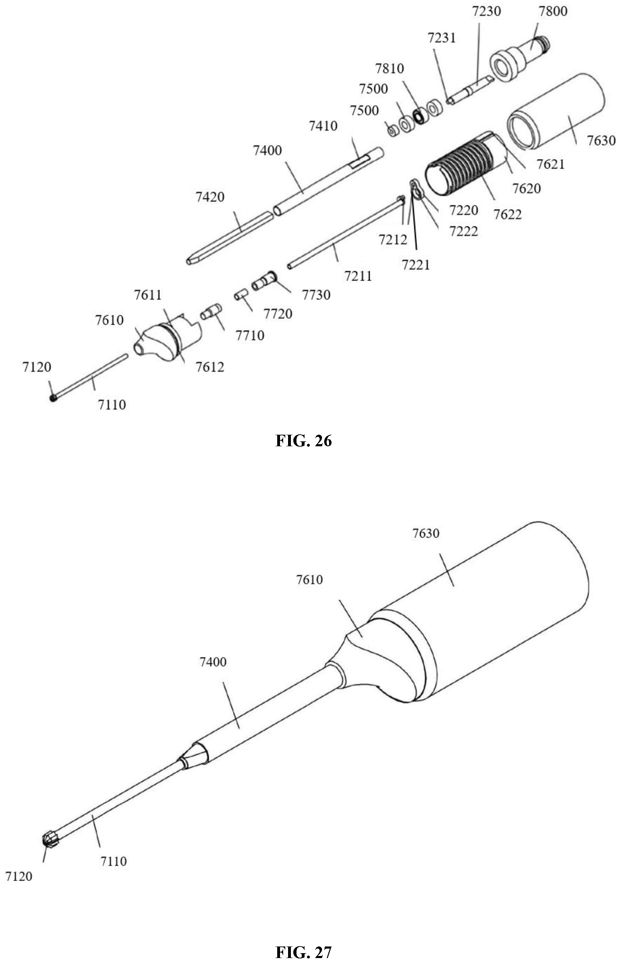

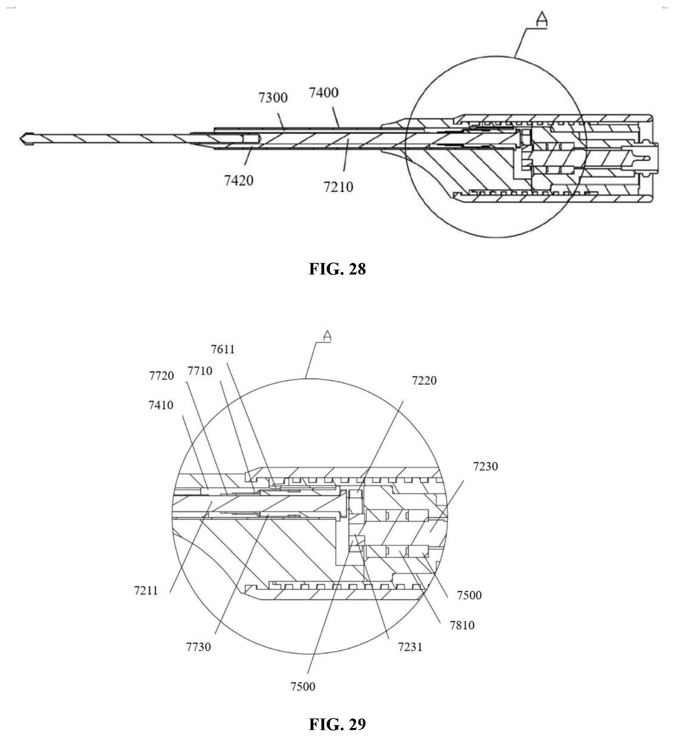

Referring to FIGS. 26 to 30, the present embodiment provides a medical reciprocating drill for orthopedic drilling. The reciprocating drill corresponds to the reciprocating cutting instrument according to the first embodiment and comprises a drill assembly, a drive assembly, and a water injection channel 7300. The drill assembly is configured to perform a reciprocating rotational motion to drill into a bone tissue. The drive assembly is configured to drive the drill assembly to perform the reciprocating rotational motion. The drive assembly comprises a reciprocating conversion rod 7210, a connecting sleeve 7220, and an input rod 7230. The reciprocating conversion rod 7210 is connected to the drill assembly. One end of the connecting sleeve 7220 is fitted over the reciprocating conversion rod 7210, and the other end is fitted over the input rod 7230. The input rod 7230 and the reciprocating conversion rod 7210 are arranged eccentrically. Rotation of the input rod 7230 drives the reciprocating conversion rod 7210 to perform the reciprocating rotational motion. The water injection channel 7300 is configured to spray water onto a drilling site on the bone tissue. An outer tube 7400 is fitted over the reciprocating conversion rod 7210, and a gap between the outer tube 7400 and the reciprocating conversion rod 7210 forms the water injection channel 7300.

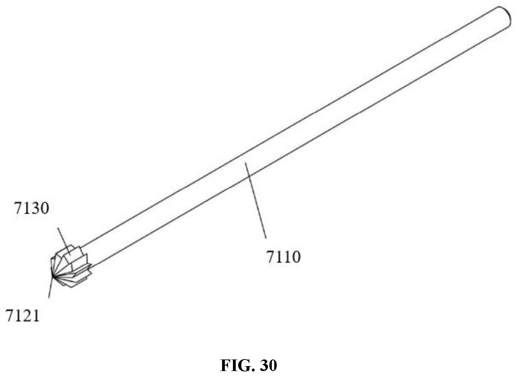

Referring to FIG. 30, the drill assembly comprises a connecting rod 7110 and a drill body 7120 fixedly connected to the connecting rod 7110. The drill body 7120 comprises a cone 7121 and a cylinder that are fixedly connected with each other, preferably integrally formed. Drill teeth 7130 are formed on side walls of both the cone 7121 and the cylinder, and a tip is formed at an end of the cone 7121 distal to the cylinder. With the above configuration, during a drilling procedure, the tip face of the cone 7121 has a smaller contact area with bone tissue, making initial penetration easier. Furthermore, the drill teeth 7130 on both the cone 7121 and the cylinder allow rapid drilling while avoiding entanglement with surrounding soft tissue when the drive assembly drives the drill assembly to perform the reciprocating rotational motion.

An end of the connecting rod 7110 proximal to the drill body 7120 is connected to the cylinder, and an end of the connecting rod 7110 distal to the drill body 7120 is inserted into the reciprocating conversion rod 7210. As the reciprocating conversion rod 7210 performs the reciprocating rotational motion, the connecting rod 7110 also performs the reciprocating rotational motion synchronously, thereby driving the drill body 7120 to perform the reciprocating rotational motion. Specifically, an insertion hole is formed inside an end of the reciprocating conversion rod 7210 proximal to the drill body 7120, and the connecting rod 7110 is inserted into the insertion hole.

The reciprocating conversion rod 7210 comprises a transmission rod 7211 and an eccentric post 7212, wherein the eccentric post 7212 is eccentrically arranged relative to the transmission rod 7211. An eccentric shaft 7231 is fixed to an end of the input rod 7230 proximal to the reciprocating conversion rod 7210 and eccentrically arranged relative to the input rod 7230. A connecting hole 7221 is formed at one end of the connecting sleeve 7220, and a mounting hole 7222 is formed at the other end. The eccentric post 7212 extends tightly through the connecting hole 7221, and the eccentric shaft 7231 is mounted in the mounting hole 7222. A bearing 7500 is mounted in the mounting hole 7222 and fixedly fitted over the eccentric shaft 7231. The input rod 7230 is connected to an external power mechanism, such as a reducer connected with a motor. When the input rod 7230 rotates, the eccentric shaft 7231 at its end performs eccentric rotation, driving the bearing 7500 in the mounting hole 7222 to slide within the mounting hole 7222 of the connecting sleeve 7220, thereby driving the connecting sleeve 7220 in left-right reciprocating circumferential movement. Power is then transmitted to the transmission rod 7211 via the eccentric post 7212, driving the transmission rod 7211 to perform the reciprocating rotational motion, thereby achieving the reciprocating rotational motion of the drill assembly and preventing unidirectional rotation of the drill body 7120.

The reciprocating drill further comprises a front case 7610, a spiral water cooling jacket 7620, and a rear case 7630. The outer tube 7400 and the reciprocating conversion rod 7210 both extend through the front case 7610. A spiral channel 7622 and a water injection groove 7621 are formed on an outer wall of the spiral water cooling jacket 7620, and the spiral channel 7622 communicates with the water injection groove 7621. A water inlet hole 7611 in communication with the interior is formed on the front case 7610. A water outlet end of the spiral channel 7622 communicates with the water inlet hole 7611, and the water inlet hole 7611 communicates with the gap between the outer tube 7400 and the reciprocating conversion rod 7210. The rear case 7630 is fitted over the spiral water cooling jacket 7620 and connected to the front case 7610. A water passage groove 7410 is formed at an end of the outer tube 7400 distal to the drill assembly and communicates with an interior of the outer tube 7400. The water inlet hole 7611 communicates with the water passage groove 7410. Specifically, the water inlet hole 7611 is located within a range corresponding to the position of the water passage groove 7410. That is, water flowing through the water inlet hole 7611 enters the gap between the outer tube 7400 and the transmission rod 7211 via the water passage groove 7410.

To enhance internal sealing of the reciprocating drill according to the present embodiment, a sealing sleeve 7710 is fitted over an end of the transmission rod 7211 distal to the drill assembly. A retaining sleeve 7720 and a bushing 7730 are further fitted between the sealing sleeve 7710 and the transmission rod 7211. Both the retaining sleeve 7720 and the bushing 7730 are tightly fitted over the transmission rod 7211 to form an interference-fit seal. The bushing 7730 is proximal to the eccentric post 7212 and also provides a structural support for the sealing sleeve 7710.

To enhance the strength and rigidity of the transmission rod 7211, a water passage tube 7420 is fitted over an end of the transmission rod 7211 proximal to the drill assembly. To better deliver water to the drilling site, one outer side wall of the water passage tube 7420 contacts an inner wall of the outer tube 7400, and a channel communicating with the exterior is formed between the other outer side wall and the inner wall of the outer tube 7400. Specifically, a facet may be formed on the other outer side wall of the water passage tube 7420 to allow water flow therethrough, or the water passage tube may be formed as an arcuate sleeve fitted over the transmission rod 7211 to provide a one-sided gap for water passage. An end of the water passage tube 7420 distal to the drill body 7120 is inserted into the front case 7610 and sealed thereto by adhesive.

To further improve sealing performance of the reciprocating drill according to the present embodiment, a circumferential slot 7612 is formed on a circumferential outer wall of the front case 7610, and a snap ring 7631 is formed on the rear case 7630. The snap ring 7631 is embedded in the circumferential slot 7612 to form a seal.

The reciprocating drill according to the present embodiment further comprises an interface housing 7800. The interface housing 7800 is located inside the spiral water cooling jacket 7620 and fitted over the input rod 7230. To enable more stable rotation of the input rod 7230, a bearing spacer 7810 is disposed inside the input rod 7230 and the interface housing 7800. Bearings 7500 are mounted at both ends of the bearing spacer 7810 and fitted over the input rod 7230.

During drilling with the reciprocating drill according to the present embodiment, driven by an external power mechanism, the input rod 7230 rotates, and the eccentric shaft at its end performs eccentric rotation, driving the bearing in the mounting hole to slide within the mounting hole 7222 of the connecting sleeve 7220, thereby driving the connecting sleeve 7220 in left-right reciprocating circumferential movement. Power is then transmitted to the transmission rod 7211 via the eccentric post 7212, driving the transmission rod 7211 to perform the reciprocating rotational motion, thereby achieving the reciprocating rotational motion of the drill assembly and preventing the drill body 7120 from easily entangling surrounding soft tissue due to unidirectional rotation.

During a drilling procedure, the reciprocating drill according to the present embodiment may inject water into the interior of the reciprocating drill from an external water injection mechanism via the water injection groove 7621 and the water then flows out through the outer tube 7400. Specifically, water in the water injection groove 7621 enters the water inlet hole 7611 via the spiral channel 7622, flows into the gap between the outer tube 7400 and the transmission rod 7211 via the water inlet hole 7611 and the water passage groove 7410, and finally flows out from an end of the outer tube 7400 proximal to the drill body 7120, flushing and cooling the drilling site to improve drilling efficiency.

In the description of the present application, it should be understood that the orientations or positional relationships indicated by the terms "upper", "lower", "front", "rear", "left", "right", "vertical", "horizontal", "top", "bottom", "inner", "outer" and the like are based on the orientations or positional relationships shown in the drawings, are merely for convenience of describing the present application and simplifying the description, and do not indicate or imply that the referred device or element must have a specific orientation, be constructed and operate in a specific orientation, and therefore should not be construed as limitations on the present application.

The specific embodiments of the present invention have been described above. It should be understood that the present invention is not limited to the above specific embodiments, and various changes or modifications that do not affect the essential content of the present invention may be made by those skilled in the art within the scope of the claims. The embodiments of the present application and the features in the embodiments may be arbitrarily combined with each other unless they conflict.

## Claims

1. A reciprocating surgical cutting instrument, **characterized in that** the reciprocating surgical cutting instrument comprises a cutter head assembly and a drive assembly, wherein the drive assembly is drivingly connected to the cutter head assembly and drives the cutter head assembly to perform a reciprocating motion.

2. A reciprocating surgical cutting instrument, **characterized in that** the reciprocating surgical cutting instrument comprises a housing, a cutter head assembly and a drive assembly, wherein the drive assembly comprises an eccentric shaft, an articulating connector and a transmission rod; the eccentric shaft, the articulating connector, the transmission rod and the cutter head assembly are all disposed in the housing, and a working end of the cutter head assembly extends out of the housing;
the articulating connector comprises a connecting frame and a swing rod, the articulating connector is rotatably mounted in the housing, one end of the eccentric shaft extends into the connecting frame and is rotatably engaged with the connecting frame, the swing rod is hinged to one end of the transmission rod, and the other end of the transmission rod is drivingly connected to the cutter head assembly;
rotation of the eccentric shaft drives the articulating connector to reciprocally swing about a hinge axis thereof, and the swing rod on the articulating connector drives the transmission rod to perform a linear reciprocating motion along an axis of the transmission rod.

3. The reciprocating surgical cutting instrument according to claim 2, **characterized in that** the cutter head assembly comprises a saw blade, a cutter head seat and a connecting rod; one end of the connecting rod is coaxially fixed to the transmission rod, the other end of the connecting rod is coaxially fixed to the cutter head seat; the saw blade is fixedly mounted on an end of the cutter head seat distal to the connecting rod, and extends out of the housing.

4. The reciprocating surgical cutting instrument according to claim 3, **characterized in that** a mounting sleeve is disposed at an end of the housing distal to the cutter head assembly, the eccentric shaft and the articulating connector are both disposed in the mounting sleeve, a first water injection channel is formed between the mounting sleeve and the housing, a second water injection channel is formed between the connecting rod and the housing, a third water injection channel is formed between the cutter head seat and the housing, and the first, second and third water injection channels are in sequential fluid communication.

5. The reciprocating surgical cutting instrument according to claim 3, **characterized in that** a support sleeve is disposed between the housing and the connecting rod, a sealing sleeve is fitted over an end of the support sleeve distal to the articulating connector, and the sealing sleeve seals a junction between the support sleeve and the connecting rod.

6. The reciprocating surgical cutting instrument according to claim 3, **characterized in that** a body of the saw blade has a uniform-thickness structure, an axial mounting groove is formed in the cutter head seat, and one end of the saw blade is embedded and mounted in the axial mounting groove;
a rectangular facet is formed on an outer surface of the cutter head seat, a connecting sleeve is disposed between the cutter head seat and a case, and a third water injection channel is formed between the rectangular facet of the cutter head seat and the connecting sleeve.

7. A reciprocating surgical cutting instrument, **characterized in that** the reciprocating surgical cutting instrument comprises a housing, a cutter head assembly and a drive assembly, wherein the cutter head assembly comprises an oscillating blade and an oscillating blade seat, the oscillating blade is fixedly connected to the oscillating blade seat, the oscillating blade and/or the oscillating blade seat are hinged to the housing, and an end of the oscillating blade distal to the oscillating blade seat extends out of the housing;
the drive assembly comprises an eccentric shaft, a connecting frame is formed at an end of the oscillating blade seat distal to the oscillating blade, the eccentric shaft extends into the connecting frame and is rotatably engaged with the connecting frame, and rotation of the eccentric shaft drives the end of the oscillating blade extending out of the housing to reciprocally swing about a hinge axis.

8. The reciprocating surgical cutting instrument according to claim 7, **characterized in that** the drive assembly further comprises a rotating shaft and a transmission shaft, the rotating shaft is coaxially fixed to the transmission shaft, and an end of the transmission shaft distal to the rotating shaft is coaxially fixed to an end of the eccentric shaft distal to the oscillating blade seat.

9. The reciprocating surgical cutting instrument according to claim 8, **characterized in that** a mounting sleeve is disposed at an end of the housing distal to the cutter head assembly, the rotating shaft is disposed in the mounting sleeve, a structural support sleeve is disposed between the transmission shaft and the housing, and a sealing sleeve is disposed between the transmission shaft and the structural support sleeve;
a connecting sleeve is connected to an end of the structural support sleeve distal to the rotating shaft, and the eccentric shaft is rotatably disposed in the connecting sleeve;
water injection channels in fluid communication are formed between the mounting sleeve, the structural support sleeve, the connecting sleeve and the housing, respectively, and openings of the water injection channels are located at an end of the connecting sleeve proximal to the cutter head assembly.

10. A reciprocating surgical cutting instrument, **characterized in that** the reciprocating surgical cutting instrument comprises a housing, a cutter head assembly and a drive assembly, wherein the cutter head assembly comprises a trephine, the drive assembly comprises an eccentric shaft, a transmission swing rod and a central transmission shaft, the transmission swing rod comprises a connecting frame and a transmission rod, the connecting frame is located at one axial end of the transmission rod, the eccentric shaft extends into the connecting frame and is rotatably engaged with the connecting frame, and an end of the transmission rod distal to the connecting frame is coaxially fixed to one end of the central transmission shaft;
the other end of the central transmission shaft is fixedly connected to the trephine, and a working portion of the trephine extends out of the housing;
rotation of the eccentric shaft drives the transmission swing rod to reciprocally swing, and the transmission swing rod drives the trephine to reciprocally rotate via the central transmission shaft.

11. The reciprocating surgical cutting instrument according to claim 10, **characterized in that** a mounting sleeve is disposed at an end of the housing distal to the cutter head assembly, the eccentric shaft is disposed in the mounting sleeve, a structural support sleeve is disposed between the central transmission shaft and the housing, and a sealing sleeve is disposed between the central transmission shaft and the structural support sleeve;
a fail-safe sleeve is disposed between the central transmission shaft and the housing;
a water injection channel is formed between the mounting sleeve and the housing, a through hole communicating an inner cavity of the central transmission shaft with the water injection channel is formed in the central transmission shaft, and a water injection hole is formed inside the trephine and communicates with the inner cavity of the central transmission shaft.

12. The reciprocating surgical cutting instrument according to claim 11, **characterized in that** a cutter head of the trephine is a hollow ring, and a weight-reduction window is formed in a body portion of the trephine.

13. A reciprocating surgical cutting instrument, **characterized in that** the reciprocating surgical cutting instrument comprises a housing, a cutter head assembly and a drive assembly, wherein the drive assembly comprises an eccentric shaft, a transmission collar, an eccentric transmission shaft and a connecting shaft, the cutter head assembly comprises a grinding/milling cutter head, the transmission collar comprises a first ring portion and a second ring portion, the first ring portion is fitted over the eccentric shaft and is rotatably engaged with the eccentric shaft, the eccentric transmission shaft extends into the second ring portion and is rotatably engaged with the second ring portion, and an end of the eccentric transmission shaft distal to the transmission collar is drivingly connected to the connecting shaft;
an end of the connecting shaft distal to the eccentric transmission shaft is drivingly connected to the grinding/milling cutter head;
rotation of the eccentric shaft drives the eccentric transmission shaft to rotate via the transmission collar, thereby driving the grinding/milling cutter head to reciprocally rotate via the connecting shaft.

14. The reciprocating surgical cutting instrument according to claim 13, **characterized in that** grinding teeth are formed on both an end face and a side face of the grinding/milling cutter head, and a connecting arcuate surface is formed between the grinding teeth on the end face and the grinding teeth on the side face of the grinding/milling cutter head;
a central portion of the end face of the grinding/milling cutter head is concave.

15. The reciprocating surgical cutting instrument according to claim 13, **characterized in that** a mounting sleeve is disposed at an end of the housing distal to the cutter head assembly, the eccentric shaft and the transmission collar are both disposed in the mounting sleeve, the eccentric transmission shaft is drivingly connected to the connecting shaft via a structural tube, a structural support sleeve is disposed between the structural tube and the housing, and a sealing sleeve is disposed between the central transmission shaft and the structural support sleeve;
a first water injection channel is formed between the mounting sleeve and the housing, a second water injection channel is formed between the structural support sleeve and the housing, a third water injection channel is formed between the structural tube and the housing, the first, second and third water injection channels are in sequential fluid communication, and a water outlet of the third water injection channel is located at an end of the structural tube proximal to the grinding/milling cutter head.

16. A surgical reciprocating grinding instrument, **characterized in that** the surgical reciprocating grinding instrument comprises a cutter head and a drive conversion assembly configured to drive the cutter head to perform a reciprocating rotational motion, wherein the drive conversion assembly comprises a transmission shaft, an input shaft and an adapter; one end of the transmission shaft is connected to the cutter head, a first eccentric post is fixed to the other end of the transmission shaft, the adapter comprises a yoke and a conversion link, the first eccentric post is mounted to one end of the conversion link, one prong of the yoke is connected to the other end of the conversion link, a first bearing is mounted in a fork opening of the yoke, a second eccentric post is fixed to one end of the input shaft, the second eccentric post is eccentrically arranged relative to the input shaft and is inserted into the first bearing, and the yoke cooperates with the first bearing and the conversion link to convert a unidirectional rotational motion of the input shaft into a reciprocating rotational motion of the transmission shaft.

17. The surgical reciprocating grinding instrument according to claim 16, **characterized in that** the surgical reciprocating grinding instrument further comprises a case, wherein the transmission shaft extends through the case, a pin is inserted into the case, and the yoke is rotatably mounted on the pin;
insertion holes are formed at opposite ends of the conversion link, a connecting post is fixed to one prong of the yoke and is inserted into one of the insertion holes, and the first eccentric post is inserted into the other of the insertion holes;
the transmission shaft and the input shaft are coaxially arranged;
the reciprocating grinding instrument further comprises an interface housing and a housing sleeve, the input shaft extends through the interface housing, one end of the housing sleeve is fitted over the case, and the other end is fitted over the interface housing;
a second bearing is mounted in the interface housing and fitted over an end of the input shaft proximal to the second eccentric post;
an outer cutter tube is fitted over the transmission shaft, and an end of the outer cutter tube distal to the cutter head is inserted into the case.

18. A reciprocating drive structure for a surgical cutting instrument, **characterized in that** the reciprocating drive structure is used for connecting to and driving a cutting instrument assembly to reciprocally rotate, and comprises:
an eccentric rocker rod fixedly connected to the cutting instrument assembly and configured to drive the cutting instrument assembly to reciprocally rotate, the eccentric rocker rod comprising a first connecting rod and a first eccentric rod that are fixedly connected with each other, wherein the first connecting rod is eccentrically arranged relative to the first eccentric rod;
an eccentric shaft drivingly connected to a drive motor, the eccentric shaft comprising a second connecting rod and a second eccentric rod that are fixedly connected with each other, wherein the second connecting rod is eccentrically arranged relative to the second eccentric rod and is drivingly connected to a rotating shaft of the drive motor; and
a reciprocating conversion link, wherein both the eccentric rocker rod and the eccentric shaft are mounted to the reciprocating conversion link, the first connecting rod and the second connecting rod are located on opposite sides of the reciprocating conversion link, respectively, and unidirectional rotation of the eccentric shaft drives the reciprocating conversion link to rotate, thereby driving the eccentric rocker rod to reciprocally rotate.

19. The reciprocating drive structure for a surgical cutting instrument according to claim 18, **characterized in that** the second connecting rod is connected to a transmission shaft, and the transmission shaft is drivingly connected to the rotating shaft of the drive motor;
the reciprocating drive structure further comprises a cutter head seat, the first connecting rod extends through the cutter head seat and is capable of reciprocally rotating in the cutter head seat while maintaining a fixed axis, and the first eccentric rod is located outside the cutter head seat;
a first support tube is fitted over an end of the first connecting rod proximal to the cutting instrument assembly, and one end of the first support tube is inserted into the cutter head seat;
a bearing housing is disposed outside the eccentric shaft, and a first bearing is mounted on the second connecting rod and installed in the bearing housing.

20. A surgical cutting instrument, **characterized in that** the surgical cutting instrument comprises the reciprocating drive structure and the cutting instrument assembly according to any one of claim 18 or 19, and further comprises a water injection tube extending through the cutter head seat, wherein a water outlet end of the water injection tube is located on an end face of the cutter head seat facing the cutting instrument assembly;
the surgical cutting instrument further comprises an adapter sleeve assembly, a second support tube and a spiral water cooling jacket, one end of the second support tube is connected to the cutter head seat, the other end of the second support tube is connected to the adapter sleeve assembly, the spiral water cooling jacket is located inside the adapter sleeve assembly, a spiral channel is formed in an outer wall of the spiral water cooling jacket and communicates with the water injection tube, and the water injection tube extends through the second support tube;
the adapter sleeve assembly comprises an adapter tube and a case mounted on the adapter tube, the spiral water cooling jacket is located inside the case, the spiral channel in the outer wall of the spiral water cooling jacket and an inner wall of the case form part of a water injection channel, and the transmission shaft extends through the adapter tube.

21. A medical reciprocating drill for orthopedic drilling, **characterized in that** the medical reciprocating drill comprises:
a drill assembly configured to perform a reciprocating rotational motion to drill into a bone tissue;
a drive assembly configured to drive the drill assembly to perform the reciprocating rotational motion, the drive assembly comprising a reciprocating conversion rod, a connecting sleeve and an input rod, wherein the reciprocating conversion rod is connected to the drill assembly, one end of the connecting sleeve is fitted over the reciprocating conversion rod, the other end is fitted over the input rod, the input rod and the reciprocating conversion rod are eccentrically arranged, and rotation of the input rod drives the reciprocating conversion rod to perform the reciprocating rotational motion; and
a water injection channel configured to spray water onto a drilling site on the bone tissue, wherein an outer tube is fitted over the reciprocating conversion rod, and a gap between the outer tube and the reciprocating conversion rod forms the water injection channel.

22. The medical reciprocating drill for orthopedic drilling according to claim 21, **characterized in that** the drill assembly comprises a connecting rod and a drill body fixedly connected to the connecting rod, and the drill body comprises a cone and a cylinder that are fixedly connected with each other;
drill teeth are formed on side walls of both the cone and the cylinder, an end of the connecting rod proximal to the drill body is connected to the cylinder, and an end of the connecting rod distal to the drill body is inserted into the reciprocating conversion rod;
the reciprocating conversion rod comprises a transmission rod and an eccentric post, the eccentric post is eccentrically arranged relative to the transmission rod, an eccentric shaft is fixed to an end of the input rod proximal to the reciprocating conversion rod and is eccentrically arranged relative to the input rod, a connecting hole is formed at one end of the connecting sleeve, a mounting hole is formed at the other end of the connecting sleeve, the eccentric post extends through the connecting hole, the eccentric shaft is mounted in the mounting hole, a bearing is mounted in the mounting hole and fitted over the eccentric shaft;
the reciprocating drill further comprises a front case, a spiral water cooling jacket and a rear case, the outer tube and the reciprocating conversion rod both extend through the front case, a spiral channel is formed in an outer wall of the spiral water cooling jacket, a water inlet hole communicating with an interior is formed in the front case, a water outlet end of the spiral channel communicates with the water inlet hole, the water inlet hole communicates with the gap between the outer tube and the reciprocating conversion rod, and the rear case is fitted over the spiral water cooling jacket and connected to the front case;
a water passage groove is formed at an end of the outer tube distal to the drill assembly and communicates with an interior of the outer tube, and the water inlet hole communicates with the water passage groove;
a sealing sleeve is fitted over an end of the transmission rod distal to the drill assembly, and a retaining sleeve is further fitted between the sealing sleeve and the transmission rod;
a water passage tube is fitted over an end of the transmission rod proximal to the drill assembly, one outer side wall of the water passage tube contacts an inner wall of the outer tube, and a channel communicating with an exterior is formed between the other outer side wall and the inner wall of the outer tube;
a circumferential slot is formed in a circumferential outer wall of the front case, a snap ring is formed on the rear case, and the snap ring is embedded in the circumferential slot to form a seal.
